(19) Europäisches Patentamt
European Patent Office
Office européen des brevets

(11) Publication number: 0 299 407 B1

(12) # EUROPEAN PATENT SPECIFICATION

(45) Date of publication of the patent specification: 24.10.90

(51) Int. Cl.[5]: C07D 231/12, A61K 31/415

(21) Application number: 88111051.4

(22) Date of filing: 11.07.88

(54) 1H-pyrazole-1-alkanamides and preparation useful as antiarrhythmic agents.

(30) Priority: 13.07.87 US 72490
13.06.88 US 206246

(43) Date of publication of application: 18.01.89 Bulletin 89/3

(45) Publication of the grant of the patent: 24.10.90 Bulletin 90/43

(84) Designated Contracting States: AT BE CH DE ES FR GB GR IT LI NL SE

(56) References cited:
CH-A- 630 083
DE-A- 3 126 083
DE-A- 3 424 586
US-A- 4 182 895

(73) Proprietor: STERLING DRUG INC., 90 Park Avenue, New York New York(US)

(72) Inventor: Bailey, Denis Mahlon, 4 Johnny Place, East, Greenbush New York(US)

(74) Representative: Baillie, Iain Cameron et al, c/o Ladas & Parry Isartorplatz 5, D-8000 München 2(DE)

EP 0 299 407 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to novel 1H-pyrazole-1-alkanamides useful as antiarrhythmic agents.

U.S. Patent 4,695,566 to Heinemann et al., which is equivalent to DE-OS-3424586, published January 9, 1986, discloses as antiarrythmic agents 1H-pyrazol-3-yl (and 1H-pyrazol-5-yl)oxyacetamides of general formula

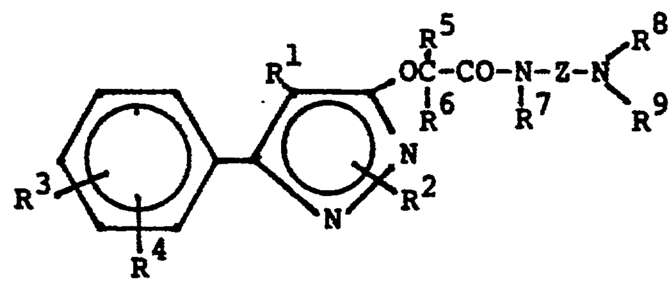

Specifically disclosed are (1) N-[2-(diethylamino)ethyl]-2-[(5-phenyl-1H-pyrazol-3-yl)oxy]acetamide, example 5, and (2) N-[3-(diethylamino)propyl]-2-[(5-phenyl-1H-pyrazol-3-yl)oxy]acetamide, example 24.

U.S. Patent 4,182,895, issued January 8, 1980, discloses as an intermediate in the synthesis of 1-amino-lower-alkyl-3,4-diphenyl-1H-pyrazoles "β-[1-(3,4-diphenyl-1H-pyrazolyl)]-N, N-dimethylpropionamide" at column 8, line 63 to 64.

The present invention relates to compounds of the formula Ia or Ib

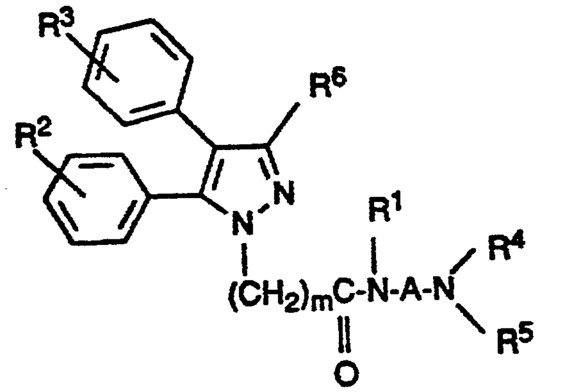

Ia

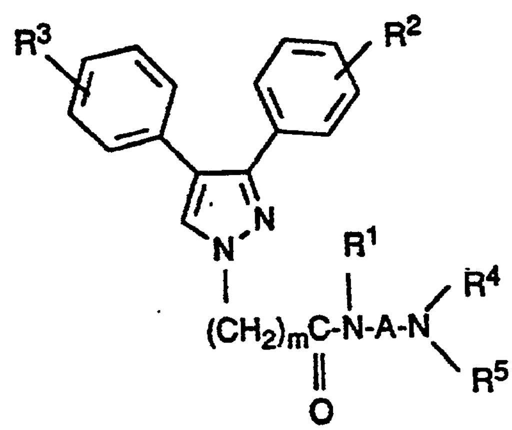

Ib .

or acid-addition salt thereof wherein $R^1$ hydrogen or lower-alkyls $R^2$ and $R^3$ are idependently hydrogen, hydroxy, lower-alkyl, lower-alkoxy or halogent $R^4$ and $R^5$ are independently hydrogen, lower-alkyl or hy-

droxy lower-alkyl or $R^4$ and $R^5$ together form an alkylene chain of four to six carbons; $R^6$ is hydrogen or hydroxy; m is one or two; A is $CH_2CH(OH)CH_2$ or $(CH_2)n$ where n is an integer from two to eight.

Lower-alkyl as used herein describes linear or branched hydrocarbon chains of four or fewer carbon atoms; lower-alkoxy as used herein describes linear or branched alkyloxy substituents containing four or fewer carbon atoms; halogen describes bromine, chlorine or fluorine.

Compositions for treating cardiac arrhythmia comprise compounds of the formula I together with pharmaceutically acceptable excipients or diluents as required. One can treat cardiac arrhythmia in a mammal by administering to said mammal an antiarrhythmicaly effective amount of a compound of formula I.

In accordance with a preferred aspect of the invention in the compounds of Formulas Ia and Ib m is one. Preferably $R^6$ is hydrogen and preferably A is $CH_2CH(OH)CH_2$ or $(CH_2)n$. In another preferred embodiment m is two and A is $(CH_2)n$

One process for preparing a compound of formule I comprises reacting a pyrazole-1-acetate or propanoate with an amine.

Another process for preparing a compound of formula I comprises reacting a 3,4 or 4,5-disubstituted pyrazole-1-acetate or propanoate with an ω-amino linear alkanol, converting the resulting alcohol to a group that is a good substrate for nucleophilic attack, and displacing the activating group with an amine.

The first process for synthesis of compounds of the invention may be outlined as shown in scheme A wherein $R^7$ is lower-alkyl.

## Scheme A

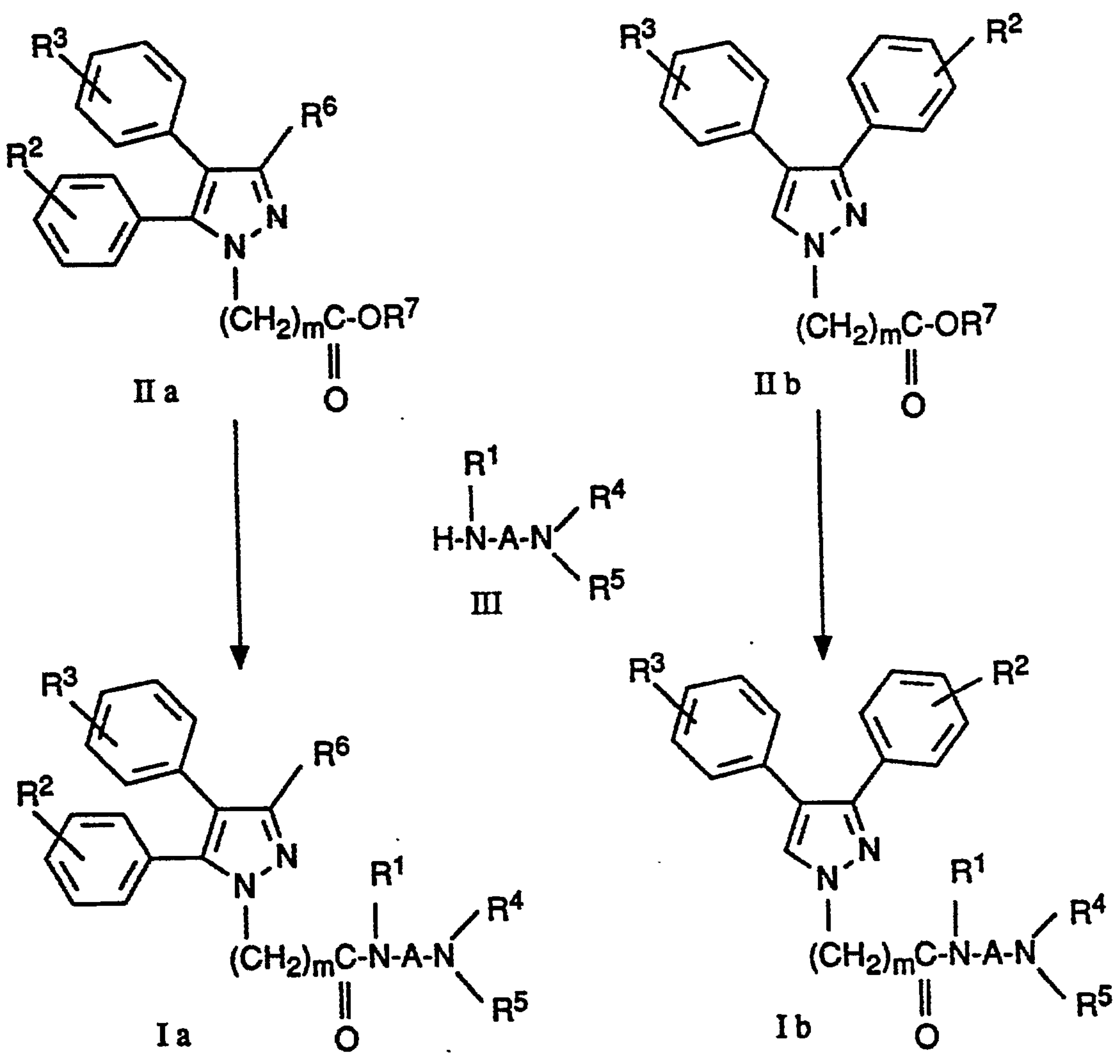
II a
II b
III
I a
I b

A lower-alkyl ester, preferably a methyl or ethyl ester, of a suitably substituted 3,4- or 4,5-diphenylpyrazole-1-alkanoic acid (II) is reacted with an excess of a primary or secondary amine of formula III

at 20° to 150°C, preferably at 90 to 150°C. When the amine is valuable, the ester II is preferably reacted with about one equivalent of the amine III in the presence of a tertiary amine, preferably diisopropylethylamine, in an inert solvent.

Alternatively, the compounds of the invention wherein A is $(CH_2)_n$ may be synthesized as outlined in Scheme B:

Scheme B

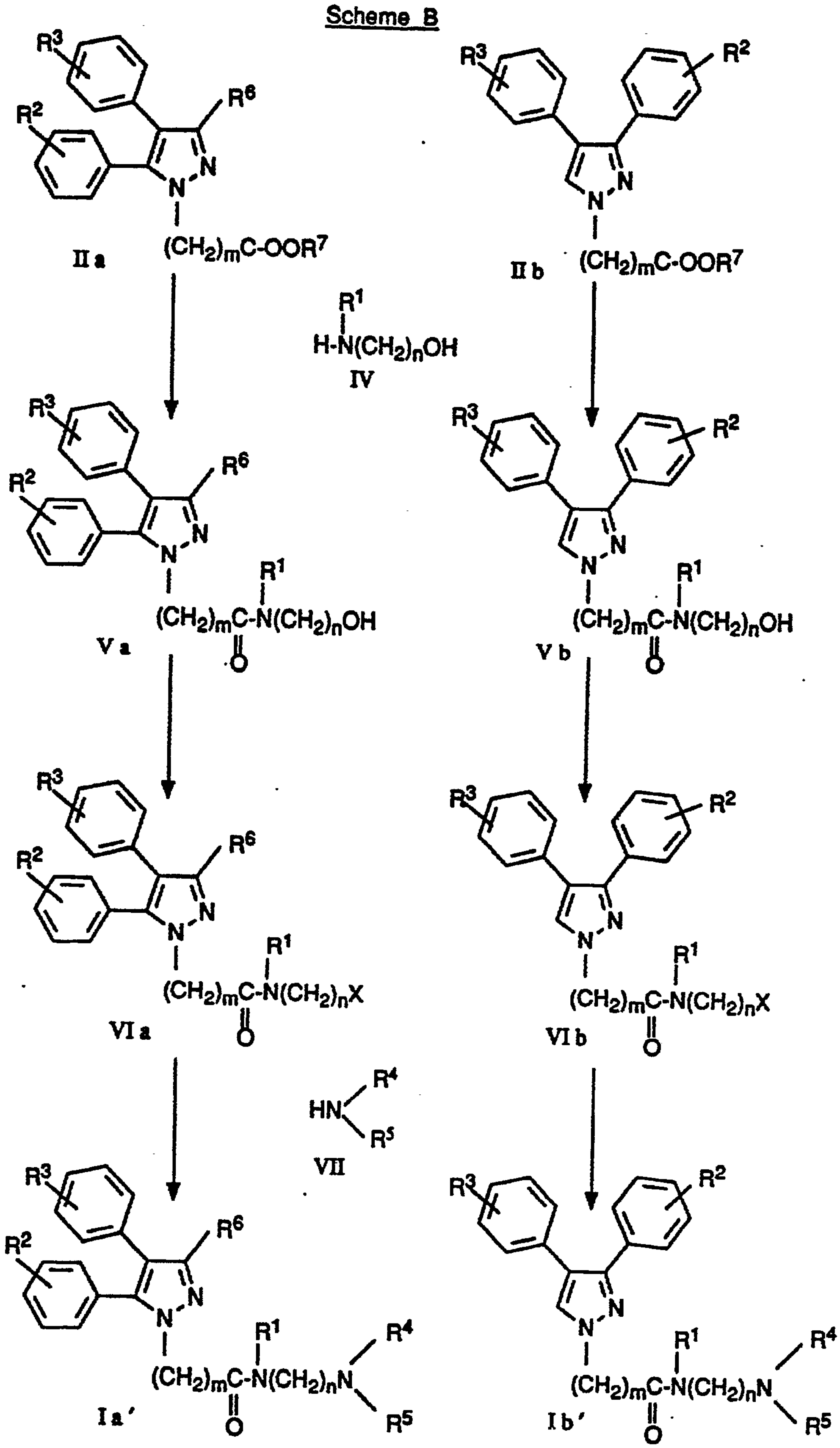

II a

II b

IV

V a

V b

VI a

VI b

VII

I a'

I b'

To summarize, the compounds of Formula Ia or Ib are prepared by reacting a compound of the formula

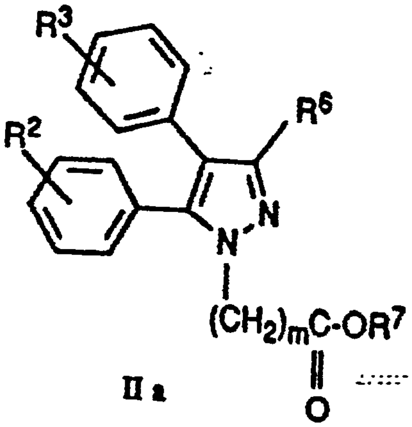

II a

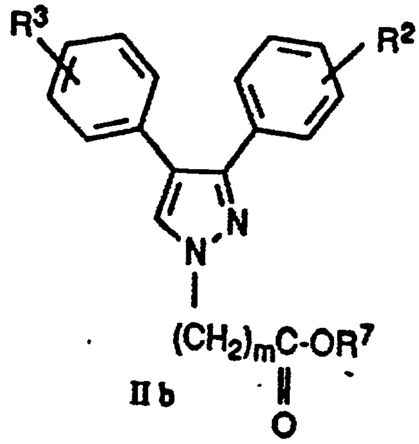

II b

wherein $R_7$ in up to $C_4$-alkyl with:

a. a compound of the Formula III

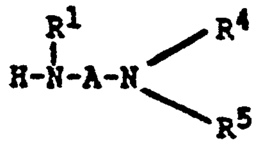

III

to prepare a corresponding compound of Formula Ia or Ib respectively or

b. a compound of the Formula IV

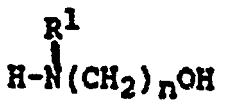

IV

to prepare a corresponding compound of the Formula Va or Vb

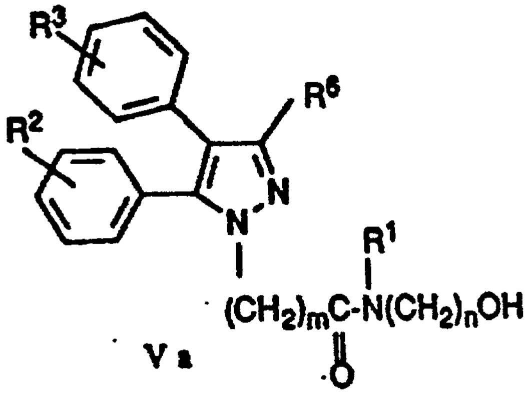

V a

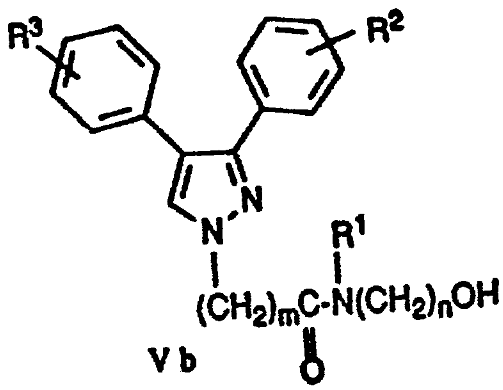

V b

respectively,

and reacting said latter compound obtained with an agent capable of converting the alcoholic function to a group suitable for nucleophilic attack to prepare a corresponding compound of Formula VIa or VIb,

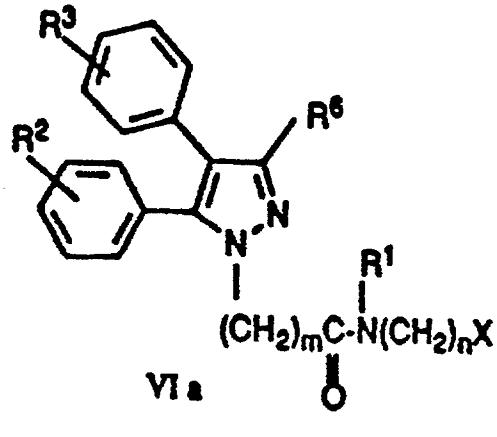

VI a

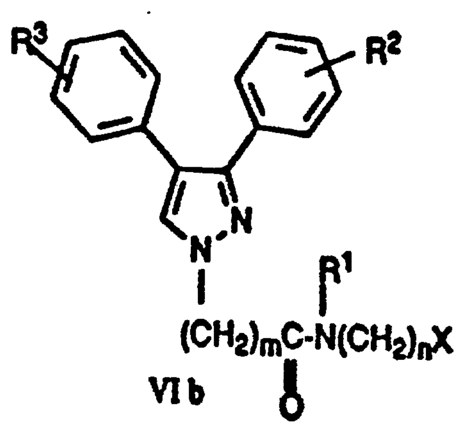

VI b

respectively, and reacting the latter compound with a compound of Formula VII

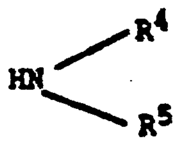

VII

so as to obtain the corresponding compound of formula Ia or Ib, respectively, where A is (CH2)n, wherein R1, R2, R3, R4, R5, m and n are as defined in claim 1 and x is a group subject to nucleophilice displacement, and, if desired, converting a free base obtained into an acid-addition salt thereof.

A lower-alkyl ester, preferably a methyl or ethyl ester, of a suitably substituted 3,4- or 4,5-diphenyl-1H-pyrazole-1-alkanoic acid (II) is reacted with an excess of a primary or secondary ω-aminoalkanol (IV) optionally in the presence of an external base at 20-150°C, preferably at 90-100°C, to produce an N-[ω-hydroxyalkyl]pyrazole-1-alkanamide of formula V. The hydroxyalkylalkanamide (V) is activated preferably by sulfonylation, preferably with methanesulfonyl chloride when $R^6$ is hydrogen or toluenesulfonyl chloride when $R^6$ is hydroxy, in the presence of a base/solvent such as pyridine at -20° to 20°C, preferably at 0°C, to produce an alkylalkanamide of formula VI wherein X is a group which is subject to nucleophilic displacement such as toluenesulfonate or methanesulfonate, that is, $OSO_2R_8$ where $R_8$ is alkyl or aryl, e.g., toluyl or methyl. Alternatively when $R^6$ is hydrogen, the hydroxyalkylalkanamide (V) is converted to the corresponding halide (VI, X = Cl, Br or I) by phosphorus trihalide, phosphorus pentahalide, thionyl halide or tetrahalomethane with trialkylphosphine. The group X is then displaced by reaction in the presence or absence of solvent with an appropriate primary or secondary amine (VII) at 20° to 100°C.

The ester IIa where $R^6$ is hydrogen and m is one, or IIb where m is one may be synthesized from the appropriately substituted desoxybenzoin by formylation by means of known procedures [Russell et al J. Am. Chem. Soc. 76, 5714 (1954)] followed by condensation with a hydrazinoacetic acid ester in a suitable solvent, preferably ethanol, at 20° to 100°C, preferably at 25°C. The hydrazinoacetate is preferably used in the form of a mineral acid salt from which the free hydrazine is liberated in situ by the addition of about one equivalent of a tertiary base, preferably pyridine.

Ester IIa where $R_6$ is hydrogen and m is two or IIb where m is two may be synthesized from the appropriately substituted diaryl pyrazole by a two step procedure comprising reaction with acrylonitrile in the presence of base in an inert solvent at 0°-50°C, preferably at about 20°C, followed by hydrolysis of the nitrile using methanol and hydrogen chloride in an inert solvent at 0° to 30°C and then water at 0-30°C.

When only the 4,5-diphenyl isomer of the products of formula Ia where m is one and $R^6$ is hydrogen is desired, the 4,5-diphenyl ester IIa where $R^6$ is hydrogen may be synthesized from the appropriately substituted desoxybenzoin by a two-step procedure comprising reaction with N,N-dimethylformamide dimethyl acetal in an inert solvent, preferably methyl tert-butyl ether, at 20°-100°C preferably at about 55°C, followed by cyclization with a lower-alkyl ester of hydrazinoacetic acid as described above.

Other processes for the production of ester IIa where $R^6$ is hydrogen and m is two or IIb where m is two, as well as a detailed description of the determination of the identity of particular isomers, are described in U.S. Patent 4,182,895.

The ester IIa where $R^6$ is hydroxy may be synthesized from the appropriate 3,4-diphenyl-5-pyrazolone by alkylation with an ω-haloalkanoate in the presence of about one equivalent of a base, preferably potassium carbonate, in an inert solvent at 20-100°C, preferably at about 55°C.

The compounds of formula I are useful both in the free base form and the form of acid-addition salts, and both forms are within the purview of the invention. The acid-addition salts are in some cases a more convenient form for use, and in practice the use of the salt form inherently amounts to the use of the base form. The acids which can be used to prepare the acid-addition salts include preferably those

which produce, when combined with the free base, medicinally acceptable salts that is, salts whose anions are relatively innocuous to the animal organism in medicinal doses of the salts so that the beneficial properties inherent in the free base are not vitiated by side effects ascribable to the anions. In practicing our invention we found it convenient to form the hydrochloride, fumarate, toluenesulfonate, methanesulfonate, or maleate salts. However, other appropriate medicinally acceptable salts within the scope of the invention are those derived from other mineral acids and organic acids. The acid-addition salts of the basic compounds are prepared either by dissolving the free base in aqueous alcohol solution containing the appropriate acid and isolating the salt by evaporating the solution, or by reacting the free base and an acid in an organic solvent, in which case the salt separates directly, is precipitated with a second organic solvent, or can be obtained by concentration of the solution. Although medicinally acceptable salts of the basic compounds are preferred, all acid-addition salts are within the scope of my invention. All acid-addition salts are useful as sources of the free base form even if the particular salt per se is desired only as an intermediate product, as, for example, when the salt is formed only for purposes of purification or identification, or when it is used as an intermediate in preparing a medicinally acceptable salt by ion exchange procedures.

The structures of the compounds of the invention were established by the mode of synthesis, by elemental analysis, and by infrared, ultraviolet and nuclear magnetic resonance spectroscopy. The course of the reactions and the identity and homogeneity of the products were assessed by thin layer chromatography (TLC) or gas-liquid chromatography (GLC). The starting materials are either commercially available or may be prepared by procedures well known in the art.

In the following procedures melting points are given in degrees C and are uncorrected. The abbreviation THF stands for tetrahydrofuran, DMF stands for N,N-dimethylformamide and Ac stands for the acetyl residue, $CH_3CO$.

## Example 1A

Ethyl 4,5-diphenyl-1H-pyrazole-1-acetate

A slurry of 200 g (0.89 mol) of formyldesoxybenzoin and 138 g (0.89 mol) of ethyl hydrazinoacetate hydrochloride in 2 L of ethanol were stirred at room temperature and 72 mL (0.89 mol) of pyridine was added dropwise. The reaction was stirred at room temperature and progress was assessed by periodic TLC using 3% acetic acid, 25% acetone and 72% toluene on silica gel. When, after the addition of a further 3 mL of pyridine over the course of 18 hours, the reaction was judged complete by TLC, the solvent was stripped in vacuo and the residue slurried in ethyl acetate. The ethyl acetate solution was filtered free of solid impurity, washed with water then saturated sodium chloride solution and dried over magnesium sulfate. The ethyl acetate was stripped to a reddish oil which was triturated in pentane to yield 156 g of solid. The product was recrystallized carefully from ether-pentane to yield 44.6 g of ethyl 4,5-diphenyl-1H-pyrazole-1-acetate, mp 79-81°C. By repeated careful crystallization from ether-pentane a further 99.6 g of the 4,5-diphenyl isomer may be obtained for a total yield of 144.2 g (53% yield).

## Example 1B

Ethyl 4,5-Diphenyl-1H-pyrazole-1-acetate

When only the 4,5-diphenyl isomer is desired the following procedure is preferred. A mixture of 778 g (3.96 mol) deoxybenzoin, 580 mL (4.38 mol) of N,N-dimethylformamide dimethyl acetal, and 775 mL of methyl tert-butyl ether was refluxed for 3 hours. The reaction mixture was cooled on ice to 0-5°C. The precipitated solid was collected by filtration, the filter cake washed with 250 mL of cold methyl tert-butyl ether twice and dried in vacuum chamber at 65°C to afford 913 g (92%) of 3-(dimethylamino)-1,2-diphenyl-2-propen-1-one, mp 128-133°C. A slurry of 913 g (3.64 mol) of 3-(dimethylamino)-1,2-diphenyl-2-propen-1-one in 3.4 L of absolute ethanol was treated with 618 g (4 mol) of ethyl hydrazinoacetate hydrochloride in one portion. The mixture was stirred at room temperature for 1 hour, filtered through diatomaceous earth, and the filtrate treated with 7 L of 50% aqueous ethanol with stirring. Cooling of the resultant solution to 0-5°C provided a white solid which was collected by filtration, washed with 250 mL of cold 50% ethanol twice and dried in vacuum at 40°C to provide 970 g (87%) of ethyl 4,5-diphenyl-1H-pyrazole-1-acetate, mp 76-80°C, containing none of the 3,4-diphenyl isomer detectable by GLC.

## Example 2

Ethyl 3,4-diphenyl-1H-pyrazole-1-acetate

Chromatography of the mother liquors from Example 1A on silica gel using 1:1 chloroform-hexane provided up to 20% of the 3,4-diphenyl isomer. The 3,4-diphenyl isomer (Example 2) may be distinguished from the 4,5-diphenyl isomer (Example 1) by its higher Rf on TLC. An analytical sample may be obtained by distillation at 0.2 mm, boiling range 186-189°C.

## Example 3

Ethyl 4,5-bis(4-fluorophenyl)-1H-pyrazole-1-acetate and ethyl 3,4-bis(4-fluorophenyl)-1H-pyrazole-1-acetate

Following the procedure of Example 1, 19.7 g (0.076 mol) of 1,2-bis(4-fluorophenyl)-3-hydroxy-2-propen-1-one, 11.8 g (0.076 mol) of ethyl hydrazinoacetate hydrochloride and 6.3 mL (0.078 mol) of pyridine were reacted at room temperature to produce 19.9 g of the mixed 4,5- and 3,4-diphenyl isomers. The isomers were separated by high pressure liquid chromatography on silica gel eluting with 97% toluene, 3% ethyl acetate. The peak with k′=2.0 yielded 1.8 g of the 3,4-diphenyl isomer, mp 98-99°C, and the peak with k′=4.0 yielded 11.16 g of the 4,5-diphenyl isomer, mp 83-84°C.

## Example 4

Methyl 3,4-diphenyl-1H-pyrazole-1-propanoate and Methyl 4,5-diphenyl-1H-pyrazole-1-propanoate

Thirty grams (0.136 mol) of 3,4 (4,5)-diphenyl-pyrazole and 11.2 mL (0.169 mol) of acrylonitrile were combined in 450 mL of methylene dichloride and 0.56 g (0.01 mol) of sodium methoxide were added. The mixture was heated with stirring under nitrogen for four hours and allowed to stand under nitrogen for 18 hours. The reaction was filtered and evaporated under vacuum below 30° C. The residue was taken up in hot absolute alcohol and cooled. The first crop of 22.7 g of damp solid was shown by NMR to consist of 30% of the 3, 4 isomer and 70% of the 4, 5 isomer. It was recrystalized a second time from about 350 mL of ethanol to yield 11 g of the pure 4,5 diphenyl isomer, mp 123-124°C. A second crop of 9 g of damp solid from the first crystalization was shown by NMR to be essentially pure 3,4 diphenyl isomer mp 82-85° C.

In an ice bath under nitrogen, hydrogen chloride gas was passed through a suspension of 100 mg (0.4 mol) of 4,5 diphenyl pyrazole-1-propionitrile and 2 mL of dry methanol for 5 to 10 minutes. Since the starting nitrile was not in solution, 2 mL of THF and 2 mL of methanol were added. The mixture was again treated with hydrogen chloride gas. The temperature shot above 20°C and the hydrogen chloride gas was turned off. The reaction was stirred at room temperature for 48 hours, and 70 mg of crystalline iminoether hydrochloride was filtered off, mp 90-91° C.

The iminoether hydrochloride was dissolved in 5 mL of methylene chloride, cooled to 0°C, and 10 drops of water were added. The reaction was stirred for a few minutes at 0°C and then for a few minutes at room temperature. The methylene chloride layer was separated and dried over magnesium sulfate, filtered and evaporated to yield 30 mg of methyl 4,5-diphenyl-1H-pyrazole-1-propanoate,mp 72-74°C.

## Example 5

Ethyl 4-(4-methoxyphenyl)-5-phenyl-1H-pyrazole-1-acetate

Following the procedure of example 1B 11 g (0.39 mol) of 3-(dimethylamino)-2-(4-methoxyphenyl)-1-phenyl-2-propene-1-one and 6.6 g (0.43 mol) of ethyl hydrazinoacetate hydrochloride were reacted in 55 mL of absolute methanol under nitrogen. After 1 1/2 hours, 11.2 g of solid product was filtered off, mp 81-84° C.

## Example 6

Ethyl 5-(4-hydroxyphenyl)-4-phenyl-1H-pyrazole-1-acetate

Following the procedure of example 1B, 15 g (0.05 mol) of 3-(dimethylamino)-1-(4-hydroxyphenyl)-2-phenyl-2-propene-1-one and 9 g(0.058 mol ) of ethyl hydrazinoacetate hydrochloride were reacted in 75 mL of absolute ethanol. After 1 1/2 hours, 14.38 g of solid product was filtered off, mp 130-135° C.

## Example 7

N-[3-(diethylamino)propyl)]-4,5-diphenyl-1H-pyrazole-1-acetamide hemihydrate

A. A mixture of 8 g (0.026 mol) of ethyl 4,5-diphenyl-1H-pyrazole-1-acetate (Example 1) and 40 mL (0.25 mol) of diethylaminopropylamine was heated on a steam bath under nitrogen for 14 hours. The excess diethylaminopropylamine was stripped in vacuo and the residue dissolved in ether. The ether solution was washed two times with water, once with saturated sodium chloride, dried over magnesium sulfate, filtered and evaporated in vacuo. The resulting white residue was dissolved in ether and a small amount of undesired material was removed by filtration after addition of some pentane. The filtrate was stripped, redissolved in ether, dried over magnesium sulfate, treated with decolorizing carbon, filtered and evaporated. The resulting residue was triturated in water, filtered and rinsed with water to yield 8.55 g (82%) of N-[3-(diethylamino)propyl]-4,5-diphenyl-1H-pyrazole-1-acetamide hemihydrate, mp 70-74°C. The product can be recrystallized from wet ether.

B. The methane sulfonate salt was prepared by dissolving 6 g of the free base in 150 mL of isopropyl alcohol and treating with one equivalent of methanesulfonic acid; mp 166-168°C.

C. The fumarate salt was prepared by dissolving 70 g of the free base in 250 mL of hot isopropyl alcohol, adding 20.8 g of fumaric acid in 100 mL of methanol, refluxing, filtering, and cooling. There was obtained 83 g of N-[3-(diethylamino)propyl]-4,5-diphenyl-1H-pyrazole-1-acetamide (E)-2-butenedioate (1:1), mp 154-156°C.

For large scale preparation of N-[3-(diethylamino)propyl]-4,5-diphenyl-1H-pyrazole-1-acetamide (E)-2-butenedioate (1:1) the following procedure was found to be superior: A solution of 2.2 kg (7.3 mol) of ethyl 4,5-diphenyl-1H-pyrazole-1-acetate in 4.3 kg (33 mol) of 3-diethylaminopropylamine was heated at 135-148°C for 1.5 hours while distilling off ethanol. Excess amine was removed by vacuum distillation; the residue was dissolved in 6L of isopropyl acetate and washed twice with 3L of water. The organic layer was concentrated to a white solid, which was dissolved in 14L of ethanol, treated with 0.96 kg (8.3 mol) of fumaric acid, and heated to 75°C to achieve a clear solution. The solid product was obtained by filtration of the cooled solution. The first crop weighed 3.1 kg (84% yield) and melted at 157-159°C; a second crop, m.p. 155-157°C, 0.36 kg (9%) was obtained upon concentration of the mother liquors.

D. The toluenesulfonate salt was prepared by dissolving 70 g of the free base in 250 mL of hot isopropyl alcohol, treating with 34 g of p-toluenesulphonic acid monohydrate in 100 mL of isopropyl alcohol, filtering and cooling. There was obtained 96.8 g of the toluenesulfonate salt, mp 126-129°C.

E. The maleate salt was prepared by dissolving 70 g the free base in 250 mL of isopropyl alcohol and 1 L of isopropyl acetate, adding 20.8 g of maleic acid, refluxing, cooling and stripping. The resulting residue was suspended in about 600 mL of ethyl acetate and 12.8 mL of water was added with vigorous stirring. The resulting solid was filtered off and recrystallized from 300 mL of water to yield 58.9 g of N-[3-(diethylamino)propyl]-4,5-diphenyl-1H-pyrazole-1-acetamide (Z)-2-butenedioate (1:1) sesquihydrate, mp 70-71°C.

## Example 8

### N-[3-(Dimethylamino)propyl]-4,5-diphenyl-1H-pyrazole-1-acetamide monohydrochloride hemihydrate

A mixture of 86.5 g (0.28 mol) of ethyl 4,5-diphenyl-1H-pyrazole-1-acetate of Example 1 and 340 mL (2.8 mol) of dimethylaminopropylamine was stirred on a steam bath under nitrogen for 18 hours. The excess dimethylaminopropylamine was stripped in vacuo, the residue was dissolved in 700 mL of ether and washed two times with water. On washing with saturated sodium chloride solution, the ether layer solidified. The resulting solid was filtered off and dissolved in dichloromethane, washed with saturated sodium chloride solution, and stripped. The residue was slurried in water and the solid product filtered off. After thorough drying, it was dissolved in 700 mL of absolute ethanol, treated with a slight excess of ethanolic HCl, filtered free of some undesired solids, and stripped. The residue was recrystal lized from about 800 mL of ethanol by chilling to yield 79 g of the hydrochloride ethanol solvate, mp 101-104°C, which showed a single spot on TLC on silica gel with 5% isopropyl amine in chloroform as eluant. The ethanol solvate was dissolved in about 800 mL of warm isopropyl alcohol and stripped; the process was repeated and the resulting residue was crystallized from about 350 mL of wet THF by recycling the mother liquor several times to yield 26.2 g of N-[3-(dimethylamino)propyl]-4,5-diphenyl-1H-pyrazole-1-acetamide monohydrochloride hemihydrate, mp 118-121°C.

## Example 9

### N-[2-(Dimethylamino)ethyl]-4,5-diphenyl-1H-pyrazole-1-acetamide hemihydrate

A mixture of 9 g (0.029 mol) of ethyl 4,5-diphenyl-1H-pyrazole-1-acetate of Example 1 and 31 mL (0.29 mol) of dimethylaminoethylamine was stirred on a steam bath under nitrogen for 18 hours. The excess dimethylamino ethylamine was stripped in vacuo, the residue dissolved in 300 mL of ether, treated with decolorizing carbon, filtered, and stripped to approximately 10 g of residue. This was triturated in water,

filtered, washed and recrystallized from ether to yield 7.16 g of N-[2-(dimethylamino)ethyl]-4,5-diphenyl-1H-pyrazole-1-acetamide hemihydrate, mp 85-88°C.

Example 10

N-[6-(Dimethylamino)hexyl]-4,5-diphenyl-1H-pyrazole-1-acetamide

A mixture of 8 g (0.026 mol) of ethyl 4,5-diphenyl-1H-pyrazole-1-acetate of Example 1, 4.1 g (0.028 mol) of dimethylaminohexylamine and 33.3 mL (0.19 mol) of diisopropylethylamine was stirred on a steam bath under nitrogen for 18 hours. Thin layer chromatography on silica gel with 5% isopropylamine in chloroform showed incomplete reaction. Another 1 mL of dimethylaminohexylamine was added and stirring continued on a steam bath for an additional 24 hours. The reaction was stripped invacuo, taken up in 200 mL of ether, washed two times with water, washed once with half saturated sodium bicarbonate solution, washed with water again, washed with saturated sodium chloride solution, dried over magnesium sulfate, treated with decolorizing carbon, filtered and stripped. The residue was taken up in ether and treated with a slight excess of hydrochloric acid. The hydrochloride salt was extracted into water and washed three times with ether; the water layer was chilled, and made basic with solid sodium carbonate and extracted two times with ether. The ether extracts were combined, washed once with saturated sodium chloride, dried over magnesium sulfate, treated with decolorizing carbon, filtered and stripped. The resulting residue was triturated in pentane to yield 4.14 g of N([6-(dimethylamino)hexyl]-4,5-diphenyl-1H-pyrazole-1-acetamide, mp 60-63°C.

Example 11

N-[4-(Dimethylamino)butyl]-4,5-diphenyl-1H-pyrazole-1-acetamide

By a procedure substantially similar to that of Example 10, 3.91 g of N-[4-(dimethylamino)butyl-4,5-diphenyl-1H-pyrazole-1-acetamide, mp 77-79°C., was synthesized from 8 g (0.026 mol) of ethyl 4,5-diphenyl-1H-pyrazole-1-acetate, 3.4 g of dimethylaminobutylamine (0.029 mol) and 33.3 mL (0.18 mol) of diisopropylethylamine.

Example 12

N-[2-(Diethylamino)ethyl]-4,5-diphenyl-1H-pyrazole-1-acetamide

By a procedure involving reaction conditions substantially similar to those of Example 9 and a workup substantially similar to that of Example 7, 6.46 g of N-[2-(diethylamino)ethyl]-4,5-diphenyl-1H-pyrazole-1-acetamide, mp 67-69°C., was synthesized from 10 g of ethyl, 4,5-diphenyl-1H-pyrazole-1-acetate and 46.4 mL of N,N-diethylethylenediamine. The product was recrystallized from ether.

Example 13

4,5-Diphenyl-N-[2-(1-piperidinyl)ethyl]-1H-pyrazole-1-acetamide

By a procedure substantially similar to that of Example 10, 10.57 g of 4,5-diphenyl-N-[2-(1-piperidinyl)ethyl]-1H-pyrazole-1-acetamide was synthesized from 15 g of ethyl 4,5-diphenyl-1H-pyrazole-1-acetate, 9.2 g of N-(2-aminoethyl)piperidine, and 62 mL of diisopropylethylamine. The work up did not require extraction, as the product crystallized from the cooled reaction mixture. It was recrystallized very slowly from 250 mL of 1:3 THF-ether, mp 95-97°C.

Example 14

4,5-Diphenyl-N-[3-(1-pyrrolidinyl)propyl]-1H-pyrazole-1-acetamide

By a procedure substantially similar to that of Example 9, 5.52 g of 4,5-diphenyl-N-[3-(1-pyrrolidinyl)propyl]-1H-pyrazole-1-acetamide, mp 75-79°C, was prepared from 15 g of ethyl 4,5-diphenyl-1H-pyrazole-1-acetate and 60 mL of N-(2-aminopropyl) pyrrolidine.

Example 15

4,5-Diphenyl-N-[2-(1-pyrrolidinyl)ethyl]-1H-pyrazole-1-acetamide

By a procedure substantially similar to that of Example 13, 6.67 g of 4,5-diphenyl-N-[2-(1-pyrrolidinyl)ethyl-]1H-pyrazole-1-acetamide, mp 80-84°C, was prepared from 15 g (0.049 mol) of ethyl 4,5-diphenyl-1H-pyrazole -1-acetate of Example 1, 9 mL (0.072 mol) of N-(2-aminoethyl)pyrrolidine and 62 mL (0.36 mol) of diisopropylethylamine.

Example 16

4,5-Diphenyl-N-[3-(1-piperidinyl)propyl]-1H-pyrazole-1-acetamide hemihydrate

A mixture of 15 g (0.049 mol) of ethyl 4,5-diphenyl-1H-pyrazole-1-acetate of Example 1, 10.2 g (0.02 mol) of 3-aminopropylpiperidine, and 62 mL of diisopropylethylamine was stirred on a steam bath under nitrogen for 18 hours. The excess amine was stripped in vacuo. The residue was taken up in about 300 mL of ether and washed twice with water. The product was extracted into 150 mL of cold water containing 18 mL of 10% hydrochloric acid. The water layer was washed two times with ether, treated with decolorizing carbon, filtered, chilled and made basic with solid sodium carbonate. The product was extracted into methylene dichloride, dried over sodium sulfate, filtered, and stripped to 14.7 g of solid residue. The residue was triturated in water, filtered and dried to yield 12.36 g of 4,5-diphenyl-N-[3-(1-piperdinyl)propyl]-1H-pyrazole-1-acetamide hemihydrate, mp 81-85°C.

Example 17

N-[3-(Diethylamino)propyl]-4,5-bis(4-fluorophenyl)-1H-pyrazole-1-acetamide

By a procedure substantially similar to that of Example 9, 8 g (0.023 mol) of ethyl 4,5-bis(4-fluorophenyl)-1H-pyrazole-1-acetate of Example 3 and 34.3 mL (0.22 mol) of diethylaminopropylamine were reacted to produce 4.45 g of N-[3-(diethylamino)propyl]-4,5-bis(4-fluorophenyl)-1H-pyrazole-1-acetamide. The solid product after trituration in water collapsed to a non-crystalline, white, waxy solid upon drying.

Example 18

N-[3-(Diethylamino)propyl]-N-methyl-4,5-diphenyl-1H-pyrazole-1-acetamide (E)-2-butenedioate (1:1)

A mixture of 28.7 g (0.09 mol) of ethyl 4,5-diphenyl-1H-pyrazole-1-acetate of Example 1, 19.7 g (0.13 mol) of diethylaminopropylmethylamine, and 94 mL (0.54 mol) of diisopropylethylamine was refluxed under nitrogen for seven days. The reaction was stripped and applied to a silica gel column using dichloromethane. Impurities were eluted with dichloromethane followed by 1.25% isopropylamine in dichloromethane. The product was eluated with 1.25% isopropylamine in chloroform. The 8 g of product was dissolved in 40 mL of warm acetone and treated with 2.3g of fumaric acid. Upon cooling, there was obtained 9.87 g of N-[3-(diethylamino)propyl]-N-methyl-4,5-diphenyl-1H-pyrazole-1-acetamide (E)-2-butenedioate (1:1), mp 139-141°C.

Example 19

N-[3-[bis(1-methylethyl)amino]propyl]-4,5-diphenyl-1H-pyrazole-1-acetamide

By a procedure substantially similar to that of Example 10, 2.49 g of N-[3-[bis(1-methylethyl)amino]propyl]-4,5-diphenyl-1H-pyrazole-1-acetamide, mp 83-84°C, was synthesized from 12 g (0.039 mol) of ethyl 4,5-diphenyl-1H-pyrazole-1-acetate, 6.2 g (0.039 mol) of diisopropylaminopropylamine, and 35 mL of diisopropylethylamine.

Example 20

N-[3-(Diethylamino)-2-hydroxypropyl]-4,5-diphenyl-1H-pyrazole-1-acetamide

By a procedure substantially similar to that of Example 10, 8.9 g of N-[3-(diethylamino)-2-hydroxypropyl]-4,5-diphenyl-1H-pyrazole-1-acetamide, mp 70-72°C, was synthesized from 15 g (0.049 mol) of ethyl 4,5-diphenyl-1H-pyrazole-1-acetate, 10 g (0.69 mol) of 1-amino-3-diethylamino-2-propanol, and 57 mL of diisopropylethylamine.

Example 21

N-[3-(Dimethylamino)propyl]-3,4-diphenyl-1H-pyrazole-1-acetamide (E)-2-butenedioate (1:1)

A mixture of 8.35 g (0.027 mol) of ethyl 3,4-diphenyl-1H-pyrazole-1-acetate of Example 2 and 33.4 mL (0.26 mol) of dimethylaminopropylamine was stirred on a steam bath under nitrogen for 18 hours. The reaction was stripped to dryness, the residue dissolved in dichloromethane, washed twice with water and once with saturated sodium chloride solution. The product was extracted into about 100 mL of cold water containing about 12 mL of 10% HCl. The water layer was made basic with solid sodium carbonate and the product extracted into methylene dichloride, dried over sodium sulfate and stripped. The product was purified by chromatography on silica gel eluting with 5% triethylamine in chloroform. The purified product was crystallized from acetone as the fumurate salt and was recrystallized from ethanol to yield 4.47 g of N-[3-(dimethylamino)propyl]-3,4-diphenyl-1H-pyrazole-1-acetamide (E)-2-butenedioate (1:1), mp 163-168°C.

Example 22

N-[3-(Diethylamino)propyl]-3,4-diphenyl-1H-pyrazole-1-acetamide

By a procedure substantially similar to that of Example 21, omitting the formation of the fumarate salt, 9 g of N-[3-(diethylamino)propyl]-3,4-diphenyl-1H-pyrazole-1-acetamide was prepared from 10 g (0.033 mol) of ethyl 3,4-diphenyl-1H-pyrazole-1-acetate and 50 mL (0.32 mol) of diethylaminopropylamine. The product was an oil.

Example 23

3,4-Diphenyl-N-[2-(1-piperidinyl)ethyl-]1H-pyrazole-1-acetamide

By a procedure substantially similar to that of Example 13, 14.0 g of 3,4-diphenyl-N-[2-(1-piperidinyl)ethyl]1H-pyrazole-1-acetamide was prepared from 16.4 g (0.054 mol) of ethyl 3,4-diphenyl-1H-pyrazole-1-acetate, 10 g (0.07 mol) of 2-aminoethylpiperidine, and 65 mL (0.37 mol) of diisopropylethylamine. The product was not recrystallized but was triturated in ether, mp 120-124°C. A second polymorph of mp 142-144°C may be obtained by recrystallizing from ethyl acetate.

Example 24

3,4-Diphenyl-N-[3-(1-piperidinyl)propyl]-1H-pyrazole-1-acetamide (E)-2-butenedioate (1:1)

By a procedure substantially similar to that of Example 21, 8.56 g of 3,4-diphenyl-N-[3-(1-piperidinyl) propyl]-1H-pyrazole-1-acetamide (E)-2-butanedioate (1:1), mp 179-180°C, was prepared from 20 g (0.065 mol) of ethyl 3,4-diphenyl-1H-pyrazole-1-acetate, 13.7 g (0.096 mol) of 3-aminopropylpiperidine, and 78 mL (0.45 mol) of diisopropylethylamine.

Example 25

N-[2-(Diethylamino)ethyl]-3,4-diphenyl-1H-pyrazole-1-acetamide

A mixture of 10 g (0.033 mol) of ethyl 3,4-diphenyl-1H-pyrazole-1-acetate, 6.9 mL (0.049 mol) of diethylaminoethylamine and 39 mL of diisopropylethylamine was stirred on a steam bath under nitrogen for 18 hours. The reaction was stripped, the residue taken up in about 300 mL of ether and washed twice with water. The ether layer was extracted twice with a total of 150 mL of cold water containing 20 mL of 10% HCl. The combined water extracts were washed once with ether, cooled, made basic with solid sodium carbonate, extracted several times with methylene dichloride, dried over sodium sulphate, treated with decolorizing carbon, filtered and stripped. The oily red residue was crystallized from ether to yield 6.86 g of N-[2-(diethylamino)ethyl-3,4-diphenyl-1H-pyrazole-1-acetamide, mp 80-83°C.

Example 26

N-(3-Aminopropyl)-4,5-diphenyl-1H-pyrazole-1-acetamide (1:4) hydrate

A mixture of 7.96 g (0.026 mol) of ethyl 3,4-diphenyl-1H-pyrazole-1-acetate and 19.2 g (0.26 mol) of 1,3-diaminopropane in 13 mL of ethanol was stirred at 84-87°C for 3 hours and the solvent removed in vacuo. The resulting solid was chromatographed on 34 g of silica gel eluting with 1% isopropylamine in chloroform and a gradient from 0-30% methanol. The impurities came off at 2-4% methanol followed by 6.95 g of pure product. It was crystallized three times from isopropyl acetate to yield 6.09 g of N-(3-aminopropyl)-4,5-diphenyl-1H-pyrazole -1-acetamide (1:4) hydrate, mp 119-120°C.

Example 27

N-(3-Hydroxypropyl)-4,5-diphenyl-1H-pyrazole-1-acetamide

A mixture of 1.47 g (0.0048 mol) of ethyl 4,5-diphenyl-1H-pyrazole-1-acetate of Example 1, 0.72 g (0.0096 mol) of 3-amino-1-propanol and 0.024 g (0.00024 mol) of triethylamine was stirred at 100°C for two hours. Five mL of ethanol was added and the solution was poured into 20 mL of water and allowed to stand 18 hours at 5°C to crystallize. The product was filtered off, air-dried and recrystallized from 25 mL of ethyl acetate to yield 1.47 g of N-(3-hydroxypropyl)-4,5-diphenyl-1H-pyrazole-1-acetamide, mp 138-139°C.

Example 28

N-[3[(2-Hydroxyethyl)amino]propyl]-4,5-diphenyl-1H-pyrazole-1-acetamide hydrochloride

A solution of 83.6 g of N-(3-hydroxypropyl)-4,5-diphenyl-1H-pyrazole-1-acetamide of Example 27 in 250 mL of pyridine was cooled to -11°C and 29 mL (0.375 mL) of methanesulfonyl chloride was added in 5 mL of aliquots. The reaction temperature was maintained below 0° throughout the addition and for 1/2 hour after the completion of the addition. The resulting mixture was poured into 2 kg of ice and 2 L of 1M HCl, stirred vigorously for 1/2 hour, and then allowed to stand until the ice melted. The water was decanted and the oil dissolved in 1 L of dichloromethane. The dichloromethane solution was dried with magnesium sulfate, filtered and stripped. Forty-five g of the resulting oil, which contained approximately 34 g (0.083 mol) of the methanesulfonate, was mixed with 25.4 mL (0.026 mol) of ethanolamine, heated briefly to avoid crystallization and then stirred overnight at room temperature. Three hundred mL of saturated aqueous sodium bicarbonate was added and the product extracted into dichloromethane. The dichloromethane was separated, dried over magnesium sulfate and chromatographed on 1.1 kg of alumina using a gradient of 0-100% methanol in dichloromethane followed by step gradients of 1% and 2% isopropylamine in methanol and finally chloroform/methanol/isopropylamine, 50:45:5. The product came off from 75% methanol in dichloromethane through to the column wash with chloroform/methanol/isopropylamine. It was then chromatographed on silica gel using a gradient of 0-30% methanol and chloroform followed by a gradient of 0.012% to 2% isopropylamine in dichloromethane/methanol, 70:30. The pure product came off from 18% methanol in dichloromethane through to the 2% isopropylamine in dichloromethane/methanol 70:30. The resulting 13.3 g of residue was recrystallized twice from ethanol to yield 6.16 g of N-[3[(2-hydroxyethyl)amino]propyl-4,5-diphenyl-1H-pyrazole -1-acetamide hydrochloride, mp 161-163°C.

Example 29

N-[3-[Ethyl(2-hydroxyethyl)amino]propyl]-4,5-diphenyl-1H-pyrazoleacetamide (E)-2-butenedioate (1:1)

Forty-five g of the impure methanesulfonate intermediate of Example 28 was stirred at room temperature for 18 hours with 41 mL (0.037 mol) of 2-(ethylamino) ethanol in 130 mL of ethanol. The mixture was filtered, 300 mL of saturated aqueous sodium bicarbonate was added, and the product extracted into dichloromethane. The residue after removal of the methylene dichloride was chromatographed substantially in the same manner as Example 25. The resulting amorphous solid was dissolved in 50 mL of isopropanol and 3 g of fumaric acid was added; the resulting solid was dried and lixiviated in refluxing acetonitrile to yield 10.1 g of N-[3-[ethyl (2-hydroxyethyl)aminopropyl]4,5-diphenyl-1H-pyrazoleacetamide (E)-2-butanedioate (1:1),mp 157-158°C.

Example 30

4,5-Diphenyl-N-[3-(ethylamino)propyl]-1H-pyrazole-1-acetamide hydrochloride

A. A solution of 10 g (0.03 mol) of 4,5-diphenyl N-(3-hydroxypropyl)-1H-pyrazole-1-acetamide of Example 27 in 50 mL of pyridine was stirred at 0°-5°C and 5.2 mL (0.066 mol) of methanesulfonyl chloride

was added dropwise over 30 minutes. The reaction was filtered free of pyridine hydrochloride and added dropwise rapidly to 40 mL of ethylamine in 50 mL of pyridine at 0°-5°C. The reaction was heated 30 minutes on a steam bath, stripped in vacuo, dissolved in 100 mL of water, washed once with ethyl acetate, made strongly basic with aqueous KOH, extracted into ethyl acetate, dried and stripped to 9.9 g of free base which was a yellow oil. The oil was treated with 75 mL of 5N HCl in ethanol and 5 g of product was filtered off. The mother liquor was treated with about 75 mL of ether and a second crop of product obtained. The total yield of 4,5-diphenyl-N-[3-(ethylamino)propyl]-1H-pyrazole-1-acetamide hydrochloride was 6.5g, m.p. 135-137°C.

B. The dihydrochloride salt was prepared by dissolving 12 parts of the free base in 12 parts of ethanol, adding 2 equivalents (about 12 parts by volume) of 5N HCl in ethanol, 1 part of water and 5 parts of ether. The resulting solid was filtered and recrystallized from isopropyl alcohol to yield 58% of the dihydrochloride salt, mp 166-174°C.

C. The fumarate salt was prepared by dissolving part of the free base in 6 parts of isopropyl alcohol, adding one equivalent of fumaric acid, heating to complete solution, cooling and filtering off the fumarate monohydrate, mp 127-129°C in 86% yield.

## Example 31

N-[3-(Diethylamino)propyl]-4,5-diphenyl-1H-pyrazole-1-propanamide hemihydrate.

A solution of 4.8 g (0.016 mol) of methyl 4,5-diphenyl-1H-pyrazole-1-propanoate of example 4 and 48 mL of diethylaminopropylamine was heated at 120 - 125°C under nitrogen for 14 hours. The excess diethylaminopropylamine was stripped in vacuo at 85°C. The residue was dissolved in dichloromethane, an unwanted solid residue was filtered off, and the methylene chloride solution was washed twice with water and once with brine. The product was extracted into chilled dilute hydrochloric acid, and the chilled aqueous acid was made basic with solid sodium bicarbonate and then sodium carbonate. The basic water was extracted 4 times with dichloromethane, the combined washings were dried over sodium sulfate, and stripped to yield 2.4 g viscous amber oil. The oily product was dried at 40°C and 0.1 mm vacuum for 7 days to yield 1.6 g of the hemihydrate still as a viscous oil.

## Example 32

N-[3-(Diethylamino)ethyl]-4,5,-diphenyl-1H-pyrazole-1-propanamide

By a procedure substantially similar to that of example 31, except that the temperature was maintained at 105°C, 1.1 g of N-[3-(diethylamino)ethyl]-4,5-diphenyl-1H-pyrazole-1-propanamide, mp 83-84°C, was synthesized from 1.8 g (5.9 mmol) of methyl 4,5-diphenyl pyrazole propanoate of example 4 and 8.3 mL (59 mmol) of diethylaminoethylamine. The product was recrystallized from 125 mL of cyclohexane containing 0.04 mL of water.

## Example 33

N-[3-(Diethylamino)propyl]-4-(4-methoxyphenyl)-5-phenyl-1H-pyrazole-1-acetamide

A solution of 7.5 g (0.022 mol) of ethyl 4-(4-methoxyphenyl)-5-phenyl-1H-pyrazole-1-acetate of example 5 in 35 mL of diethylaminopropylamine was stirred on a steam bath under nitrogen for 14 hours. The diethylaminopropylamine was removed in vacuo and the residue taken up in 200 mL of ethyl acetate. The ethyl acetate solution was washed twice with water, once with brine, and dried over sodium sulfate. The solvent was removed in vacuo and the residue was distilled in a Kugelrohr at 250°C /0.1 mm to yield 6.39 g of N-[3-(diethylamino) propyl]-4-(4-methoxyphenyl)-5-phenyl-1H-pyrazole-1-acetamide as a clear viscous amber oil.

## Example 34

N-[3-(Diethylamino)propyl]-4-(4-hydroxyphenyl)-5-phenyl-1H-pyrazole-1-acetamide

A solution of 14 g (00.33 mol) of N-[3-(diethylamino)propyl[-4-(4-methoxyphenyl)-5-phenyl-1H-pyrazole-1-acetamide of example 33 and 0.16 mol of the sodium salt of 1 propanethiol in 375 mL of DMF was heated at 155-160°C for 2 hours. The reaction was chilled and the excess thiopropoxide was neutralized with ethanolic HCl. The DMF solution was stripped under vacuum at 30°, dissolved in methylene dichloride, and washed with aqueous sodium carbonate, then water, and finally brine. The methylene chloride was dried over sodium sulfate and the solvent removed in vacuo to yield 13.4 g of gummy product. Seven grams of the gummy product was chromatographed on a silica gel column eluted with methanol/isopropylamine/chloroform (2:3:95) to yield 5.1 g of purified product. The residue was taken up in isopropyl acetate, washed twice with water, washed once with brine, and dried over sodium sulfate. The isopropyl acetate was evaporated in vacuo, but removal of the last traces of isopropyl acetate required extensive drying under high vacuum to yield 2.5 g of product as a glass.

Example 35

N-[3-(Diethylamino)propyl-5-(4-hydroxyphenyl)-4-phenyl-1H-pyrazole-1-acetamide

A solution of 10 g (0.031 mol) of ethyl 5-(4-hydroxyphenyl)-4-phenyl-1H-pyrazole-1-acetate of example 6 in 48 mL of diethylaminopropylamine was stirred on a steam bath under nitrogen for 14 hours. The excess amine was removed in vacuo at 80°C, the residue dissolved in ethyl acetate, washed twice with water, once with brine and dried over sodium sulfate. The ethyl acetate was stripped to yield 12 g of a dark oil which was triturated in hot cyclohexane several times. The insoluble residue was combined with a small amount of a mixture of oil and crystals, which had separated from the cyclohexane on cooling, and recrystallized from ethyl acetate to yield 6.6 g of product, mp 110-113°C.

Example 36

4-(4-Bromophenyl)-N-[4-(ethylmethylamino)butyl]-5-phenyl-1H-pyrazole-1-acetamide

By a process substantially similar to that of Example 25 it is contemplated that 4-(4-bromophenyl)-N-[4-(ethylmethylamino)butyl]-5-phenyl-1H-pyrazole-1-acetamide may be synthesized from N-ethyl-N-methyl-1,4-butanediamine and ethyl 4-(4-bromophenyl)-5-phenyl-1H-pyrazole-1-acetate, which is synthesized by a process substantially similar to that of Example 1A from α-(4-bromophenyl)-β-oxobenzenepropanal.

Example 37

4-(2-Chlorophenyl)-N-[3-[(1,1-dimethylethyl)amino]propyl]-5-(4-methoxyphenyl)-1H-pyrazole-1-acetamide

By a process substantially similar to that of Example 30 it is contemplated that 4-(2-chlorophenyl)-N-[3-[(1,1-dimethylethyl)amino]propyl]-5-(4-methoxyphenyl)-1H-pyrazole-1-acetamide may be synthesized from tert-butylamine and N-[3-(methylsulfonyloxy)propyl]-4(2-chlorophenyl)-5(4-methoxyphenyl)-1H-pyrazole-1-acetamide.

Example 38

N-[7-(Diethylamino)heptyl]-4-(3-methylphenyl)-5-phenyl-1H-pyrazole-1-acetamide

By a process substantially similar to that of Example 25 it is contemplated that N-[7-(diethylamino) heptyl]-4-(3-methylphenyl)-5-phenyl-1H-pyrazole-1-acetamide may be synthesized from N,N-diethyl-1,7-heptanediamine and ethyl 4-(3-methylphenyl)-5-phenyl-1H-pyrazole-1-acetate, which is synthesized by a process substantially similar to that of Example 1B from 2-(3-methylphenyl)-1-phenylethanone.

Example 39

Ethyl 3-hydroxy-4,5-diphenyl-1H-pyrazole-1-acetate or Ethyl 1,2-dihydro-3-oxo-4,5-diphenyl-3H-pyrazol-1-acetate

The suspension of 3.3 mL (0.03 mol) of ethyl bromo-acetate, 3.9 g (0.028 mol) of anhydrous, milled potassium carbonate and 6.7 g (0.028 mol) of 1,2-dihydro-4,5-diphenyl -3H-pyrazol-3-one obtained by the method of Gruenanger and Finzi [Chemical Abstracts 58: 516f (1963)] in 67 mL of acetone was stirred at reflux for 24 hours. The solvent was removed in vacuo and the residue was chromatographed on a 45 x 300 mm silica gel column eluting with a gradient from 0 to 10% ethyl acetate in hexane, taking 75 mL fractions. Fractions 50 - 65 contained the O-alkylated product; fractions 75 - 79 provided 1.1 g of the desired N- alkylated product, mp 181 - 183°C.

Example 40

N-[3-(Diethylamino)propyl]-3-hydroxy-4,5-diphenyl-1H-pyrazole-1-acetamide or N[3-(Diethylamino) propyl]-1,2-dihydro-3-oxo-4,5-diphenyl-3H-pyrazole-1-acetamide hemihydrate

By a process substantially similar to that of example 35, 400 mg of N-[3-(diethylamino)propyl]-3-hydroxy -4,5-diphenyl-1H-pyrazole-1-acetamide or N[3-(diethylamino) propyl]-1, 2-dihydro-3-oxo-4, 5-diphenyl-3H-pyrazole-1-acetamide was prepared from 1.1 g (3.4 mol) of ethyl 3-hydroxy-4,5-diphenyl-1H-pyrazole-1-acetate of example 39 and 5.1mL (32 mol) of diethylaminopropylamine. The oily product was triturated in ether and the resulting crystals filtered off and dried. The product from ether was slurried in water and filtered to yield the hemihydrate, mp 150-152°C.

The antiarrhythmic activity of compounds of the invention was demonstrated by the following procedure.

Duncan Hartley guinea pigs (600-900 grams) of either sex were anesthetized with urethane (1.4 g/kg, i.p.) and supplemented as needed. An intravenous route for drug administration was established using microbore tubing inserted into the jugular vein. The induction of arrhythmias by aconitine hydrochloride (34 g/kg) was evaluated in control guinea pigs given 1 cc saline as an intravenous bolus 10 minutes prior to aconitine challenge.

Compounds to be tested were administered intravenously 10 minutes prior to aconitine challenge at an initial dosage of 30 mg/kg. This dosage was reduced in subsequent animals if severe cardiac rhythm disturbances persisted beyond two minutes after injection in the first guinea pig tested. All drugs were tested at the maximally tolerated dose (identified by the lack of arrhythmias in the EKG prior to aconitine challenge). Compounds were administered in saline as 1 cc bolus injections (n=5-9) at the specified doses (expressed as free base).

Time intervals between aconitine injection and the appearance of arrhythmias were determined. Specifically noted was the time until the onset of (i) the first premature ventricular contraction (PVC); (ii) the first sustained run of ventricular tachycardia consisting of 10 or more ventricular beats (VTACH); and (iii) the time until the appearance of ventricular fibrillation lasting longer than 15 seconds (VFIB). The average time and standard error of the mean until the appearance of these arrhythmias were calculated for each treatment group and compared to concurrent controls using a one-way analysis of variance. Activity was defined as a statistically significant delay in the onset of PVC, VTACH and VFIB time course compared to control values.

The following table summarizes the results obtained from the testing of representative compounds of the invention.

| Example No. | Minutes to PVC | Minutes to VTACH | Minutes to VFIB |
|---|---|---|---|
| Control | 0.97 | 1.48 | 3.96 |
| 7A | 15.7 | 16.0 | 37.1 |
| 7B | 2.8 | 10.5 | 21.1 |
| 7C | 2.8 | 3.4 | 21.8 |
| 7D | 2.4 | 2.8 | 13.2 |
| 7E | 4.9 | 7.4 | 19.8 |
| 8 | 5.9 | 7.3 | 16.2 |
| 9 | 8.2 | 8.7 | 29.0 |
| 10 | 2.6 | 3.0 | 11.7 |
| 11 | 5.0 | 5.7 | 25.7 |
| 12 | 4.1 | 5.8 | 40.1 |
| 13 | 7.9 | 10.7 | 35.4 |
| 14 | 5.1 | 6.3 | 25.7 |
| 15 | 2.9 | 3.0 | 15.5 |
| 16 | 5.2 | 15.4 | 27.6 |
| 17 | 1.0* | 2.6 | 30.0 |
| 18 | 8.4 | 11.3 | 32.0 |
| 19 | 4.5 | 6.0 | 16.5 |
| 20 | 8.4 | 10.1 | 40.7 |
| 21 | 3.9 | 5.4 | 14.5 |
| 22 | 3.9 | 20.3 | 26.5 |
| 23 | 3.6 | 9.6 | 30.0 |
| 24 | 3.6 | 8.0 | 29.0 |
| 25 | 1.9 | 2.1* | 17.7 |
| 26 | 2.1 | 2.9 | 16.7* |
| 28 | 2.1 | 11.0 | 44.1 |
| 29 | 2.9* | 5.0 | 21.0 |
| 30A | 3.9 | 13.2 | 47.7 |
| 30B | 9.0 | 10.5 | 13.8 |
| 30C | 16.3 | 22.4 | 37.0 |
| 31 | 11.6 | 21.8 | 54.7 |
| 32 | 2.5 | 2.8 | 38.6 |
| 33 | 10.9 | 15.4 | 41.5 |
| 34 | 11.6 | 38.9 | 60.0 |
| 35 | 9.4 | 38.4 | 60.0 |
| 40 | 3.0 | 6.9 | 39.0 |

*not statistically significant

The compounds of the invention can be prepared for use by conventional pharmaceutical procedures:

that is, by dissolving or suspending them or their pharmaceutically acceptable salts in a pharmaceutically acceptable vehicle, e.g., water, aqueous alcohol, glycol, oil solution or oil-water emulsion, for parenteral or oral administration;or by incorporating them in unit dosage form as capsules or tablets for oral administration either alone or in combination with conventional adjuvants or excipients, e.g., calcium carbonate, starch, lactose, talc, magnesium stearate, gum acacia, and the like.

The percentage of active component in the composition and method for treating or preventing arrhythmia can be varied so that a suitable dosage is obtained. The dosage administration to a particular patient is variable depending upon the clinician's judgement using as the criteria: the route of administration, the duration of treatment, the size and condition of the patient, the potency of the active component, and the patient's response thereto. An effective dosage amount of active component can thus be determined by the clinician considering all criteria and utilizing his best judgement on the patient's behalf.

**Claims for the Contracting States: AT, BE, CH, DE, FR, GB, IT, LI, NL, SE**

1. A compound of Formula

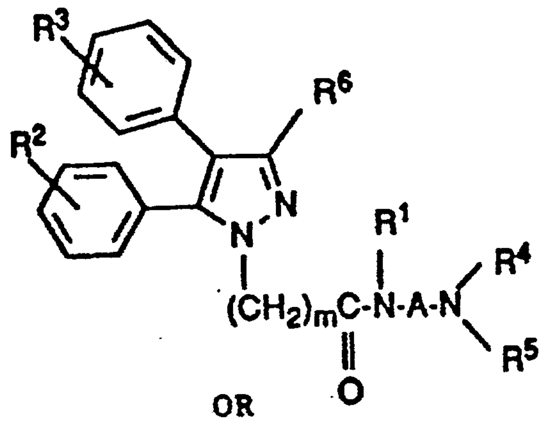

Ia

OR

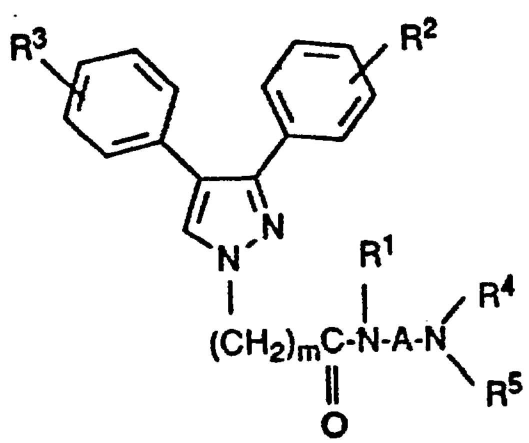

Ib

or acid-addition salt thereof, wherein $R_1$ is hydrogen or up to $C_4$-alkyl; $R_2$ and $R_3$ are independently hydrogen, hydroxy, up to $C_4$-alkyl, up to $C_4$-alkoxy or halogen: $R^4$ and $R^5$ are independently hydrogen, up to $C_4$-alkyl or hydroxy up to $C_4$-alkyl or $R^4$ and $R^5$ together form an alkylene chain of four to six carbons; $R^6$ is hydrogen or hydroxy; m is one or two; and A is $CH_2CH(OH)CH_2$ or $(CH_2)_n$ wherein n is an integer from two to eight.

2. A compound according to claim 1, wherein m is one.

3. A compound according to claim 1 or 2, wherein $R^6$ is hydrogen.

4. A compound according to any one of claims 1–3, wherein A is $CH_2CH(OH)CH_2$.

5. A compound according to any one of claims 1-3, wherein A is $(CH_2)_n$.

6. N-[3-(Diethylamino)propyl]-4, 5-diphenyl-1H-pyrazolel-acetamide or acid-addition salt thereof according to claim 5.

7. A compound according to claim 1, wherein m is two and A is $(CH_2)_n$.

8. A process for preparing a compound according to claim 1, which comprises reacting a compound of the Formula IIa or IIb

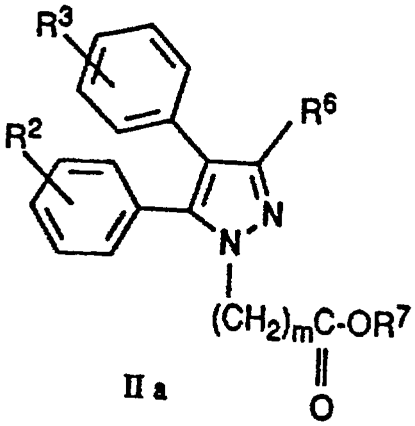

II a

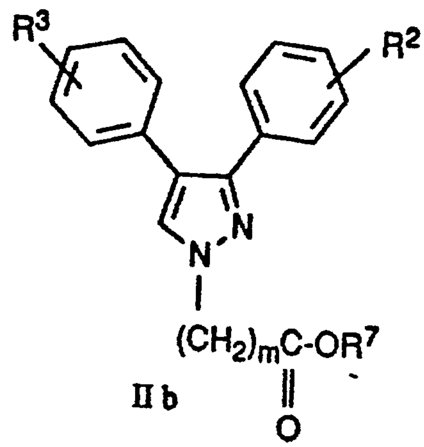

II b

wherein $R^7$ is up to $C_4$-alkyl
with:

a. a compound of the Formula III

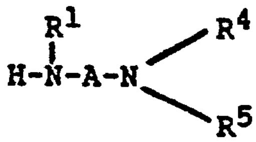

III

to prepare a corresponding compound of Formula Ia or Ib respectively or

b. a compound of the Formula IV

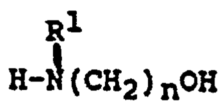

IV

to prepare a corresponding compound of the Formula Va or Vb

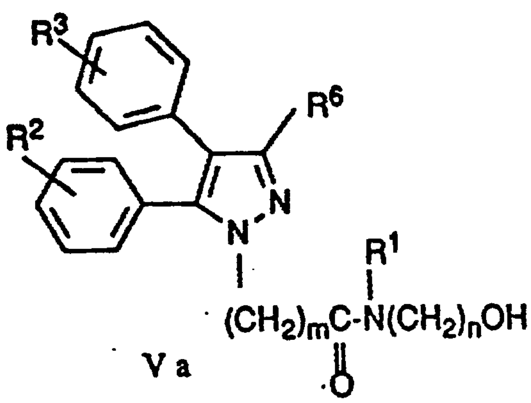

V a

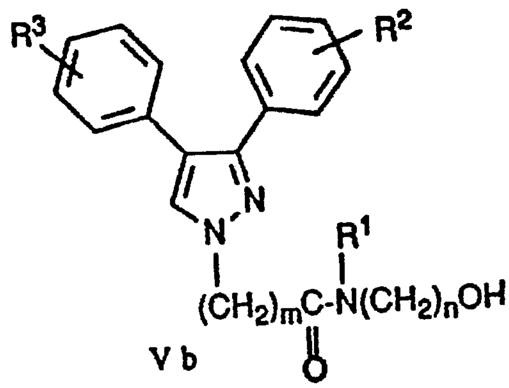

V b

respectively,

and reacting said latter compound obtained wlth an agent capablo of converting the alcoholic function to a group suitable for nucleophilic attack to prepare a corresponding compound of Formula VIa or VIb,

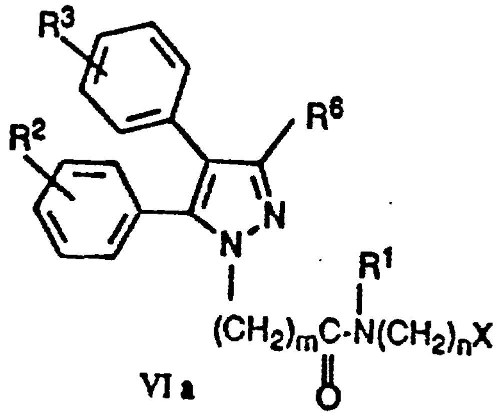
VI a

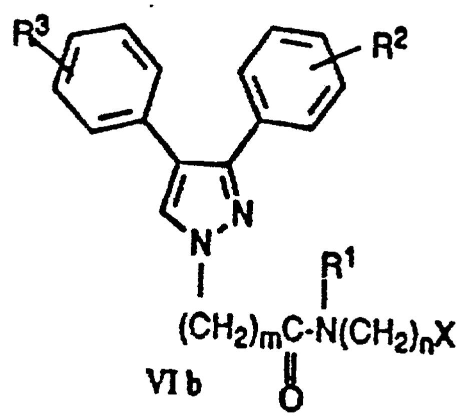
VI b

respectively, and reacting the latter compound with a compound of Formula VII

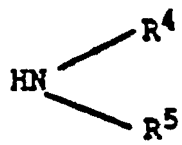
VII

so as to obtain the corresponding compound ot Formula Ia or Ib, respectively, where A is $(CH_2)_n$, wherein $R_1$, $R_2$, $R_3$, $R_4$, $R_5$, m and n are as defined in claim 1, and X is a group subject to nucleophilic displacement, and, if desired, converting a free base obtained into an acid-addition salt thereof.

9. A composition for treating cardiac arrhythmias comprising a compound according to any one of claims 1-6 present in an amount effective to treat cardiac arrhythmias together with one or more pharmaceutically acceptable excipients or diluents.

10. Use of a compound according to any of claims 1-7 for preparing a medicament for treating cardiac arrhythmias in a susceptible subject.

**Claims for the Contracting States: ES, GR**

1. A process for preparing a compound of Formula

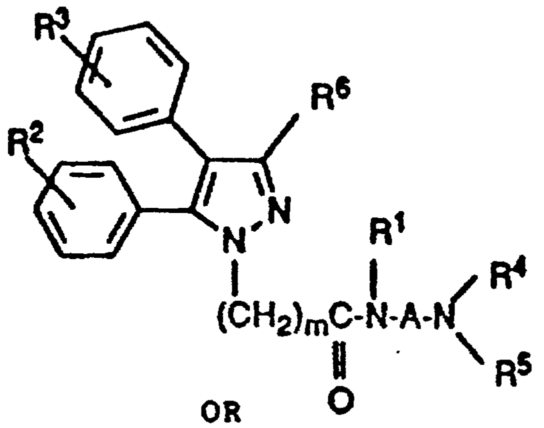

Ia

OR

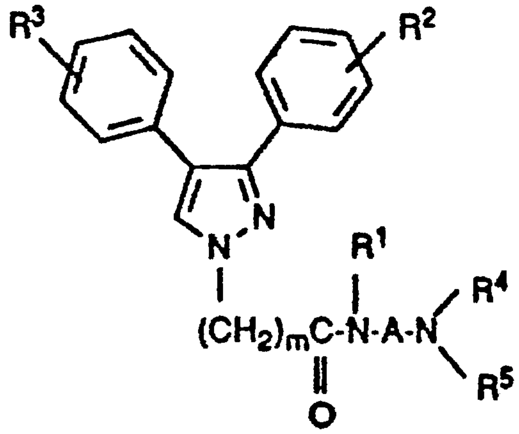

Ib

or acid-addition salt thereof, wherein $R^1$ is hydrogen or up to $C_4$-alkyl; $R^2$ and $R^3$ are independently hydrogen, hydroxy, up to $C_4$-alkyl, up to $C_4$-alkoxy $R^4$ and $R^5$ are independently hydrogen, up to $C_4$-alkyl or-hydroxy, up to $C_4$-alkyl or $R^4$ and $R^5$ together form an alkylene chain of four to six carbons; $R^6$ is hydrogen or hydroxyz; m is one or two; and A is $CH_2CH(OH)CH_2$ or $(CH_2)_n$ wherein n is an integer from two to eight characterized by reacting a compound of the Formula IIa or IIb

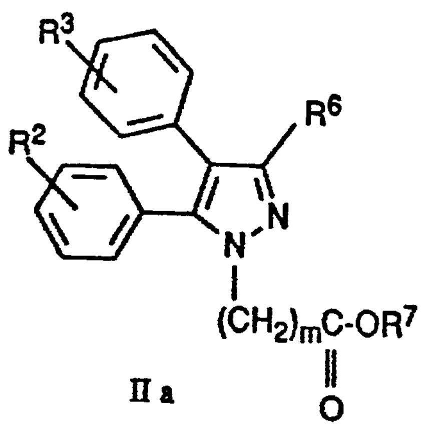

II a

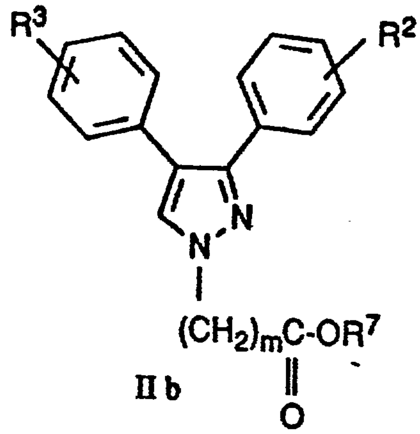

II b

wherein $R_7$ is up to $C_4$-alkyl
with:

a. a compound of the Formula III

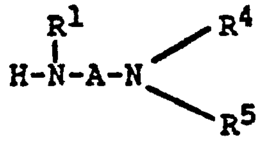

III

to prepare a corresponding compound of Formula Ia or Ib respectively or

b. a compound of the Formula IV

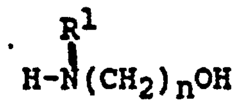

IV

to prepare a corresponding compound of the Formula Va or Vb

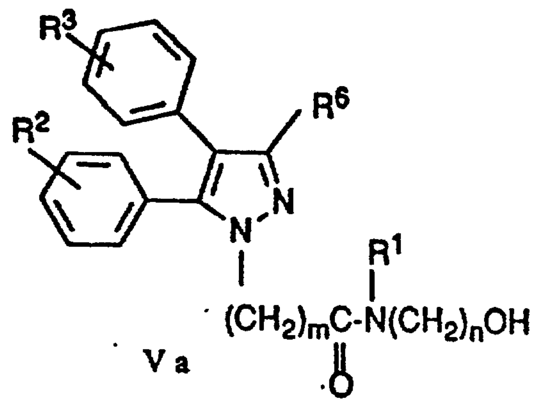

V a

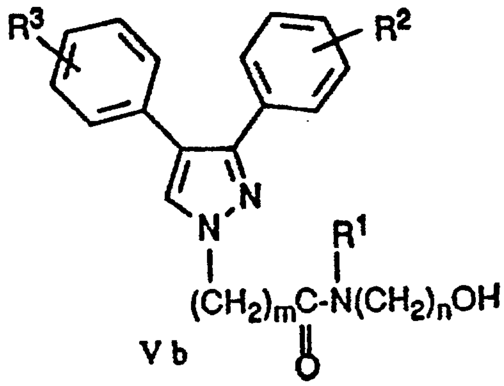

V b

respectively, and reacting said latter compound obtained with an agent capable of converting the alcoholic function to a group suitable for nucleophilic attack to prepare a corresponding compound of Formula VIa or VIb,

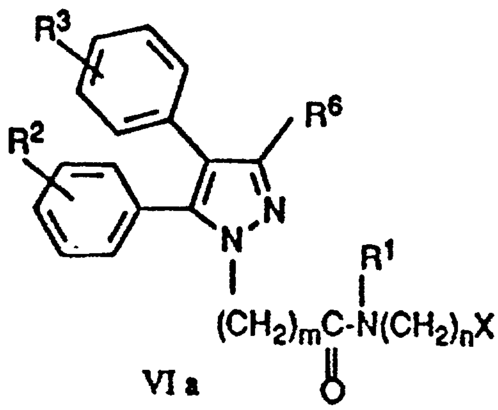

VI a

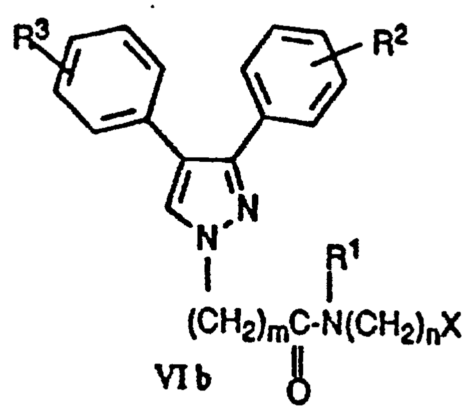

VI b

respectively, and reacting the latter compound with a compound of Formula VII

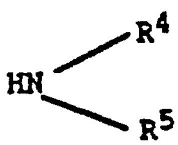

VII

so as to obtain the corresponding compound of Formula Ia or Ib, respectively, where A is $(CH_2)_n$ , wherein $R_1$, $R_2$, $R_3$, $R_4$, $R_5$, m and n are as defined above and X is a group subject to nucleophilic displacement, and, if desired, converting a free base obtained into an acid-addition salt thereof.

2. A process according to claim 1, wherein in b. the agent for converting the alcoholic function is an alkyl- or arylsulfonyl chloride, X being $OSO_2R_8$ where $R_8$ is alkyl or aryl.

3. A proces according to claim 1, wherein in b. the agent for converting the alcoholic function when $R^6$ is hydrogen is a phosphorous trihalide, phosphorous pentahalide, thionyl halide or tetrahalomethane used with a trialkyl phosphine, halo corresponding to X and being C1. Br or I.

4. A process according to any one of the preceding claims, wherein m is one.

5. A process according to any one of the preceding Claims, wherein $R^6$is hydrogen.

6. A process according to any one of the preceding claims, wherein A is $(CH_2)_n$.

7. A process according to claim 6, for preparing N -3(diethylamino)propyl]-4, 5-diphenyl-1H-pyrazole-l-acetamide which comprises reacting ethyl 4, 5-diphenyl-1H-pyrazole-1-acetate with 3-(diethylamino)propylamine.

8. A process according to claim 1, wherein m is two and A is $(CH_2)_n$

**Patentansprüche für die Vertragsstaaten: AT, BE, CH, DE, FR, GB, IT, LI, NL, SE**

1. Verbindung der Formel

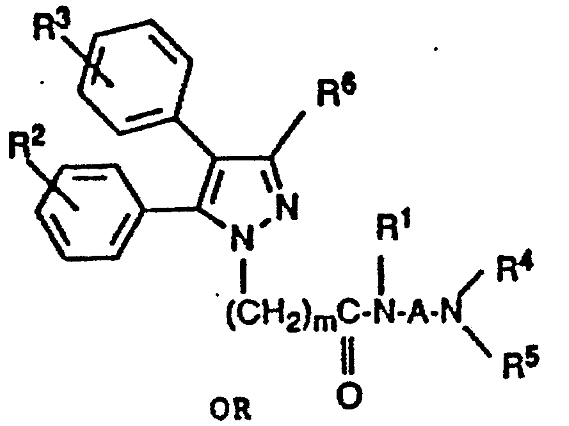

Ia

OR

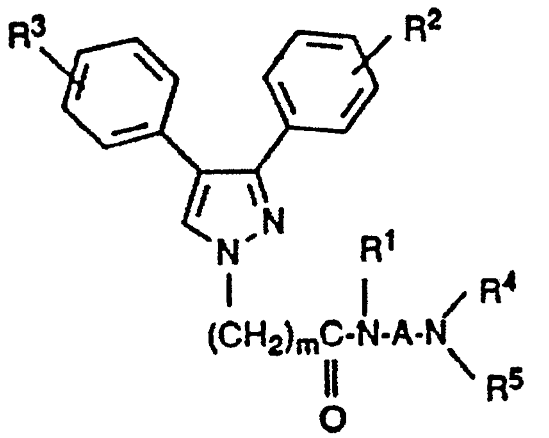

Ib

oder eines Säureadditionssalzes derselben, wobei $R^1$ Wasserstoff oder bis zu $C_4$-Alkyl ist, $R^2$ und $R^3$ unabhängig voneinander Wasserstoff, Hydroxy, bis zu $C_4$-Alkyl, bis zu $C_4$-Alkoxy oder Halogen sind, $R^4$ und $R^5$ unabhängig voneinander Wasserstoff, bis zu $C_4$-Alkyl oder Hydroxy-bis zu C4-Alkyl sind oder $R^4$ und $R^5$ zusammen eine Alkylenkette mit vier bis sechs Kohlenstoffatomen bilden, $R^6$ Wasserstoff oder Hydroxy ist, m gleich eins oder zwei ist und A $CH_2CH(OH)CH_2$ oder $(CH_2)n$ ist, wobei n eine ganze Zahl von zwei bis acht ist.

2. Verbindung nach Anspruch 1, dadurch gekennzeichnet, dass m gleich eins ist.

3. Verbindung nach Anspruch 1 oder 2, dadurch gekennzeichnet, dass $R^6$ Wasserstoff ist.

4. Verbindung nach einem der Ansprüche 1 bis 3, dadurch gekennzeichnet, dass A $CH_2CH(OH)CH_2$ ist.

5. Verbindung nach einem der Ansprüche 1 bis 3, dadurch gekennzeichnet, dass A $(CH_2)_n$ ist.

6. N-(3-(Diethylamino)propyl)-4, 5-diphenyl-1H-pyrazol-1-acetamid oder ein Säureadditionssalz desselben nach Anspruch 5.

7. Verbindung nach Anspruch 1, dadurch gekennzeichnet, dass m gleich zwei und A $(CH_2)_n$ ist.

8. Verfahren zum Erzeugen einer Verbindung nach Anspruch 1, in dem eine Verbindung der Formel IIa oder IIb

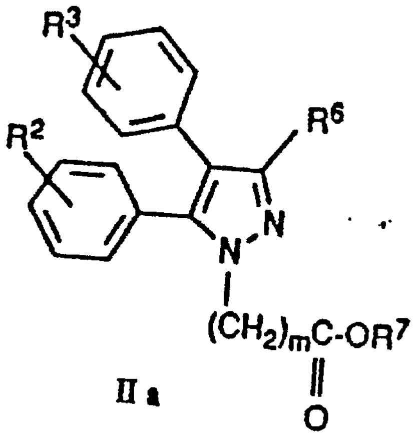

II a

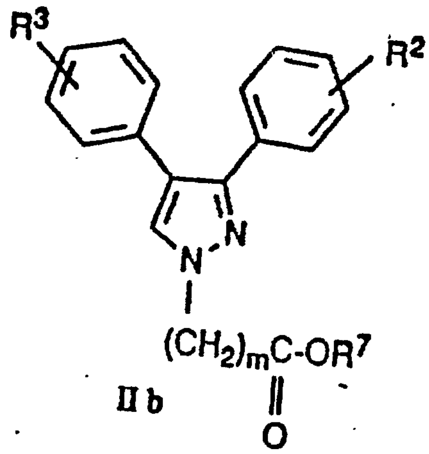

II b

in der $R_7$ bis zu $C_4$-Alkyl ist,

a. mit einer Verbindung der Formel III

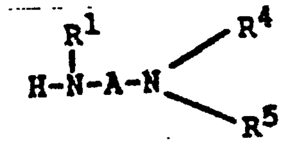

III

zu einer entsprechenden Verbindung der Formel Ia bzw. Ib umgesetzt wird oder

b. mit einer Verbindung der Formel IV

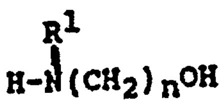

IV

zu einer entsprechenden Verbindung der Formel Va bzw. Vb

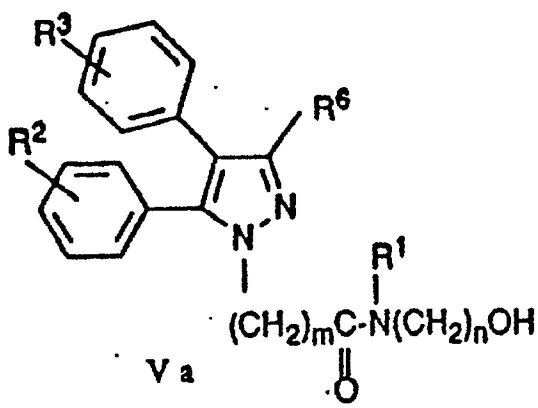

V a

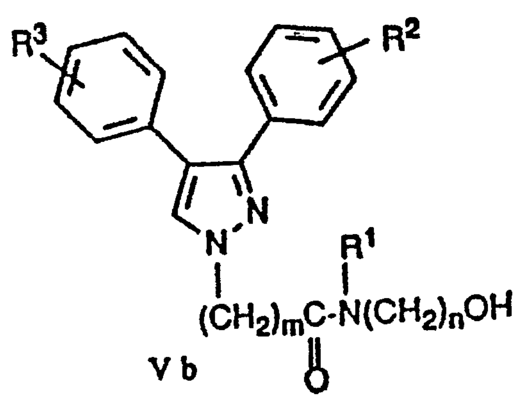

V b

umgesetzt wird, die so erhaltene Verbindung mit einem Mittel umgesetzt wird, das geeignet ist, die alkoholische Funktion in eine Gruppe umzuwandeln, die zu einem nukleophilen Angriff fähig ist, so dass eine entsprechende Verbindung der Formel VIa bzw. VIb

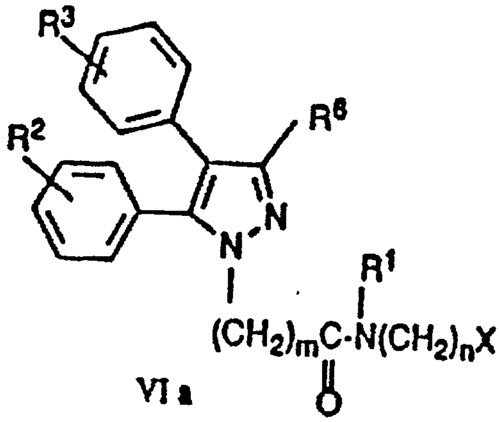

VI a

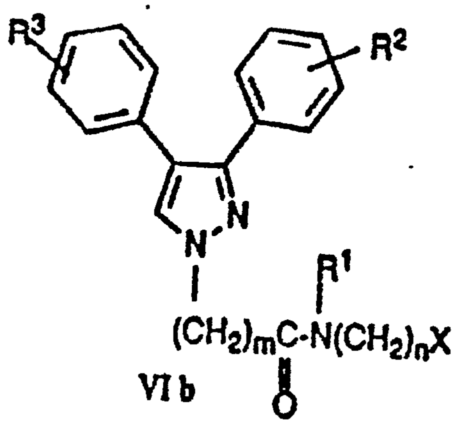

VI b

erhalten wird, und die zuletztgenannte Verbindung mit einer Verbindung der Formel VII

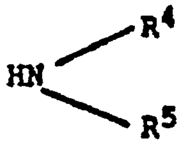

VII

zu der entsprechenden Verbindung der Formel Ia bzw. Ib umgesetzt wird, in der A $(CH_2)_n$ ist, $R_1$, $R_2$, $R_3$, $R_4$, $R_5$, m und n die vorstehend angegebenen Bedeutungen haben und X eine nukleophil verdrängbare Gruppe osz, und gegebenenfalls eine erhaltene freie Base in ein Säureadditionssalz derselben umgewandelt wird.

9. Zusammensetzung zum Behandeln von Herzrhythmusstörungen mit einer Verbindung nach einem der Ansprüche 1 bis 6 in einer für die Behandlung von Herzrhythmusstörungen wirksamen Menge zusammen mit einem oder mehreren für pharmazeutische Zwecke verwendbaren Trägerstoffen oder Verdünnungsmitteln.

10. Die Verwendung einer Verbindung nach einem der Ansprüche 1 bis 7 zum Erzeugen eines Medikaments zum Behandeln von Herzrhythmusstörungen bei einem Gefährdeten.

**Patentansprüche für die Vertragsstaaten: ES, GR**

1. Verfahren zum Erzeugen einer Verbindung der Formel

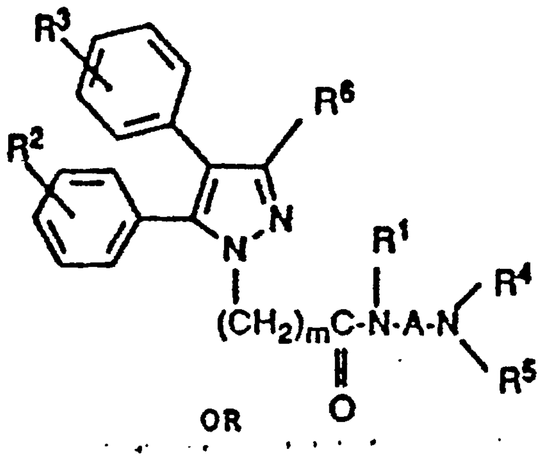

Ia

OR

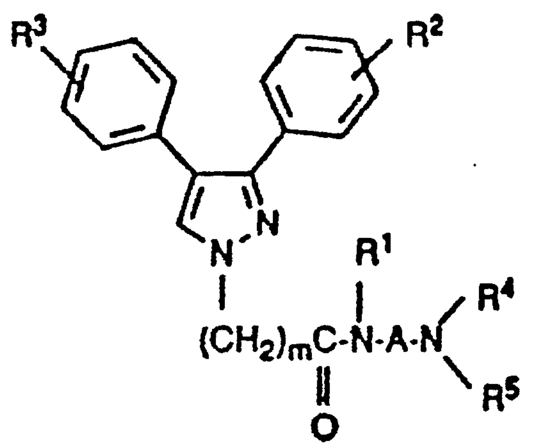

Ib

oder eines Säureadditionssalzes derselben, wobei $R^1$ Wasserstoff oder bis zu $C_4$-Alkyl ist, $R^2$ und $R^3$ unabhängig voneinander Wasserstoff, Hydroxy-, bis zu $C_4$-Alkyl, bis zu $C_4$-Alkoxy oder Halogen sind, $R^4$ und $R^5$ unabhängig voneinander Wasserstoff, bis zu $C_4$-Alkyl oder Hydroxy-bis zu $C_4$-Alkyl sind oder $R^4$ und $R^5$ zusammen eine Alkylenkette mit vier bis sechs Kohlenstoffatomen bilden, $R^6$ Wasserstoff oder Hydroxy ist, m gleich eins oder zwei ist und A $CH_2CH(OH)CH_2$ oder $(CH_2)n$ ist, wobei n eine ganze Zahl von zwei bis acht ist, dadurch gekennzeichnet, dass eine Verbindung der Formel IIa oder IIb

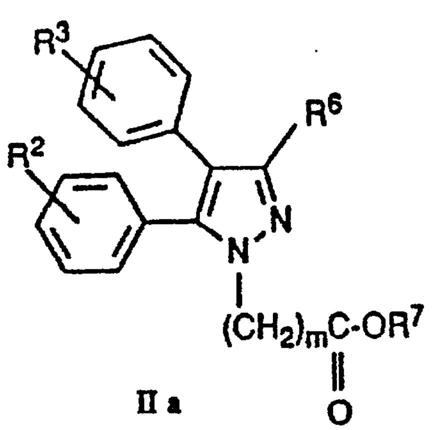

II a

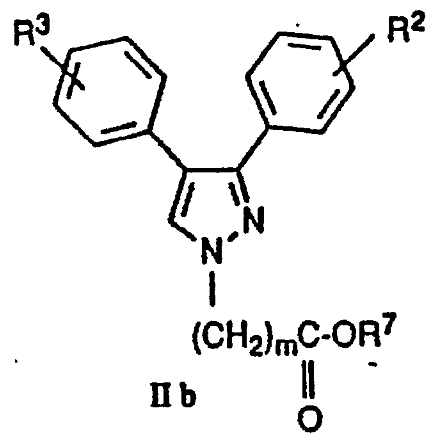

II b

in der $R_7$ bis zu $C_4$-Alkyl ist,

a. mit einer Verbindung der Formel III

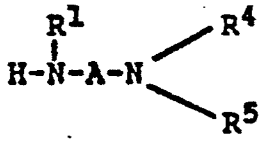

III

zu einer entsprechenden Verbindung der Formel Ia bzw. Ib umgesetzt wird oder

b. mit einer Verbindung der Formel IV

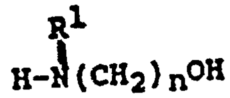

IV

zu einer entsprechenden Verbindung der Formel Va bzw. Vb

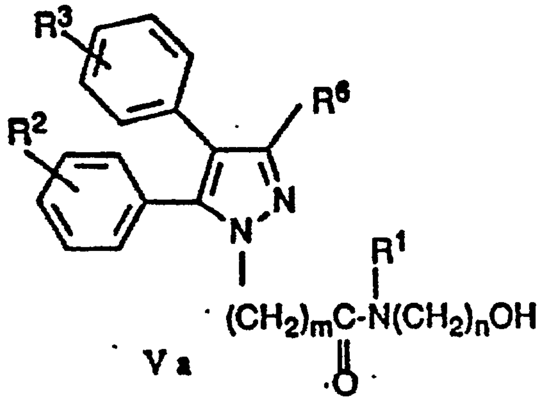

Va

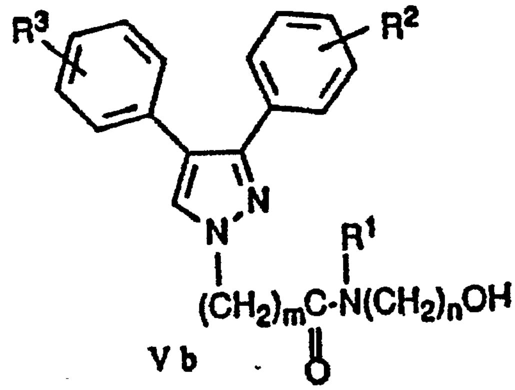

Vb

umgesetzt wird,

die so erhaltene Verbindung mit einem Mittel umgesetzt wird, das geeignet ist, die alkoholische Funktion in eine Gruppe umzuwandeln, die zu einem nukleophilen Angriff fähig ist, so dass eine entsprechende Verbindung der Formel VIa bzw. VIb

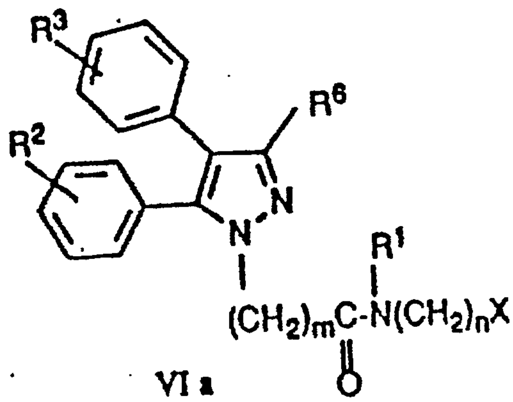

VIa

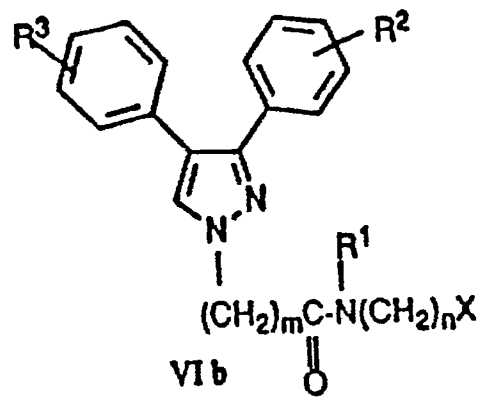

VIb

erhalten wird, und die zuletztgenannte Verbindung mit einer Verbindung der Formel VII

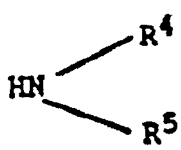

zu der entsprechenden Verbindung der Formel Ia bzw. Ib umgesetzt wird, in der A $(CH_2)_n$ ist, $R_1$, $R_2$, $R_3$, $R_4$, $R_5$, m und n die vorstehend angegebenen Bedeutungen haben und X eine nukleophil verdrängbare Gruppe osz, und gegebenenfalls eine erhaltene freie Base in ein Säureadditionssalz derselben umgewandelt wird.

2. Verfahren nach Anspruch 1, dadurch gekennzeichnet, dass das in b. verwendete Mittel zum Umwandeln der alkoholischen Funktion ein Alkyl- oder Arylsulfonylchlorid und X $OSO_2R_8$ ist, wobei $R_8$- Alkyl oder Aryl ist.

3. Verfahren nach Anspruch 1, dadurch gekennzeichnet, dass das in b. verwendete Mittel zum Umwandeln der alkoholischen Funktion für den Fall, dass $R^6$ Wasserstoff ist, ein Phosphortrihalogenid, Phosphorpentahalogenid, Thionylhalogenid oder ein Tetrahalogenmethan ist, das zusammen mit einem Trialkylphosphin verwendet wird, wobei Halogen Cl, Br oder I ist und X entspricht.

4. Verfahren nach einem der vorhergehenden Ansprüche, dadurch gekennzeichnet, dass m gleich eins ist.

5. Verfahren nach einem der vorhergehenden Ansprüche, dadurch gekennzeichnet, dass $R^6$ Wasserstoff ist.

6. Verfahren nach einem der vorhergehenden Ansprüche, dadurch gekennzeichnet, dass A $(CH_2)_n$ ist.

7. Verfahren nach Anspruch 6 zum Erzeugen von N-[3-(Diethylamino)propyl]3-4, 5-diphenyl-1H-pyrazol-1-acetamid, dadurch gekennzeichnet, dass Ethyl-4, 5-diphenyl-1H-pyrazol-1acetat mit 3-(Diethylamino)propylamin umgesetzt wird.

8. Verfahren nach Anspruch 1, dadurch gekennzeichnet, dass m gleich zwei und A $(CH2)_n$ ist.

**Revendications pour les Etats Contractants: AT, BE, CH, DE, FR, GB, IT, LI, NL, SE**

1. Composé de formule :

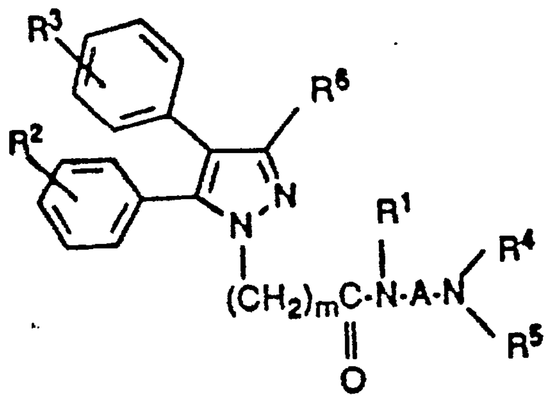

Ia

ou

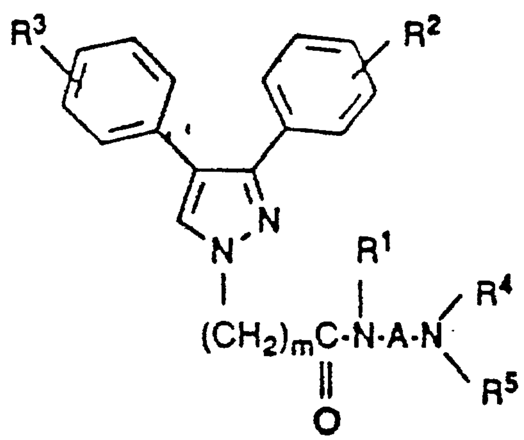

Ib

ou un sel d'addition avec un acide de celui-ci, dans lequel $R^1$ représente un atome d'hydrogène ou un groupe alkyle comportant jusqu'à 4 atomes de carbone ; $R^2$ et $R^3$ représentent indépendamment chacun un atome d'hydrogène, un groupe hydroxy, alkyle comportant jusqu'à 4 atomes de carbone, alkoxy comportant jusqu'à 4 atomes de carbone ou un atome d'halogène ; $R^4$ et $R^5$ représentent indépendamment chacun un groupe alkyle comportant jusqu'à 4 atomes de carbone ou hydroxyalkyle comportant jusqu'à 4 atomes de carbone ; ou $R^4$ et $R^5$ forment ensemble une chaîne alkylène comportant de 4 a 6 atomes de carbone ; $R^6$ représente un atome d'hydrogène ou un groupe hydroxy ; m est égal à un ou deux ; et A re-

présente un groupe $CH_2CH(OH)CH_2$ ou $(CH_2)_n$ dans lequel n est un entier de 2 à 8.

2. Composé selon la revendication 1, dans lequel m est égal à un.

3. Composé selon la revendication 1 ou 2, dans lequel $R^6$ représente un atome d'hydrogène.

4. Compose selon l'une quelconque des revendications 1 à 3, dans lequel A représente un groupe $CH_2CH(OH)CH_2$.

5. Composé selon l'une quelconque des revendications 1 à 3, dans lequel A représente un groupe $(CH_2)_n$

6. N-[3-diéthylamino)propyl]-4, 5-diphényl-1Hpyrazole-l-acétamide ou un sel d'addition avec un acide de celui-ci selon la revendication 5.

7. Composé selon la revendication 1, dans lequel m est égal à 2 et A représente un groupe $(CH_2)_n$.

8. Procédé de préparation d'un composé selon la revendication 1, dans lequel on fait réagir un composé de formule IIa ou IIb :

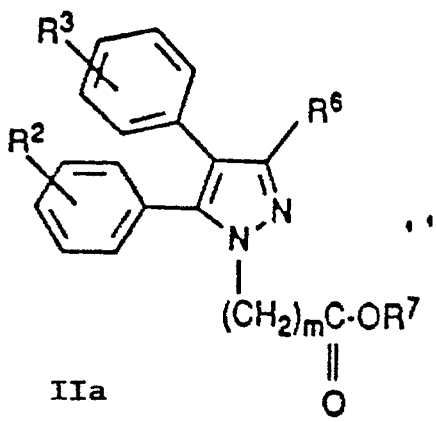

IIa

,

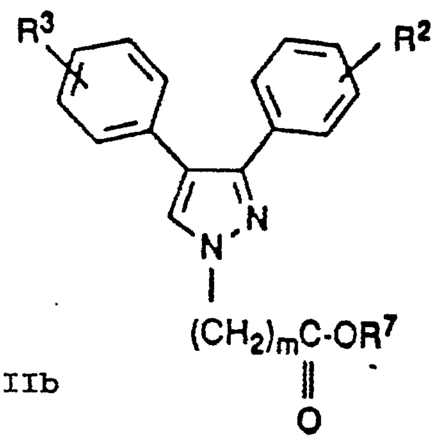

IIb

dans lesquelles $R^7$ est un groupe alkyle comportant jusqu'à 4 atomes de carbone, avec :

a. un composé de formule III :

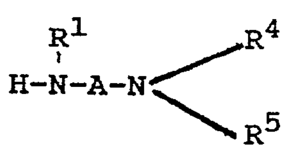

III

pour préparer un composé correspondant, respectivement de formule Ia ou Ib ; ou un composé de formule IV :

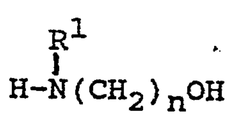

IV

pour préparer un composé correspondant, respectivement de formule Va ou Vb :

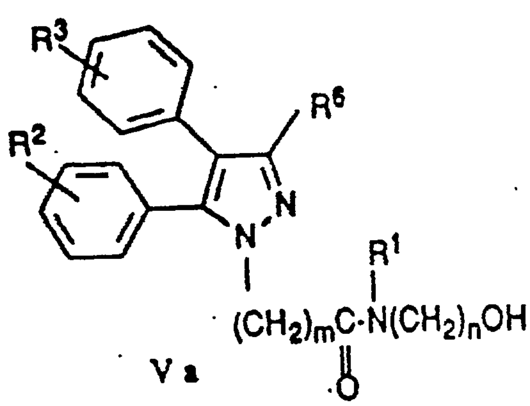

V a

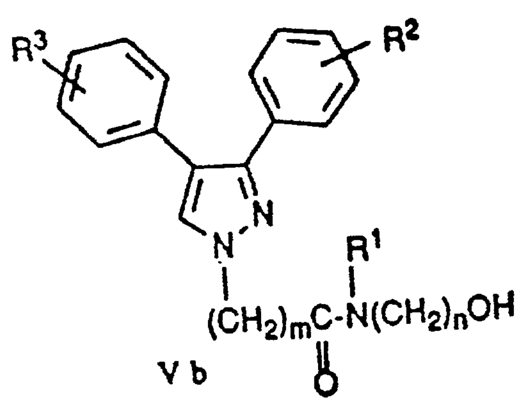

V b

et on fait réagir ce dernier composé obtenu, avec un agent capable de convertir la fonction alcool, en un

groupe approprié pour subir une attaque nucléophile, afin de préparer un composé correspondant, respectivement de formule VIa ou VIb :

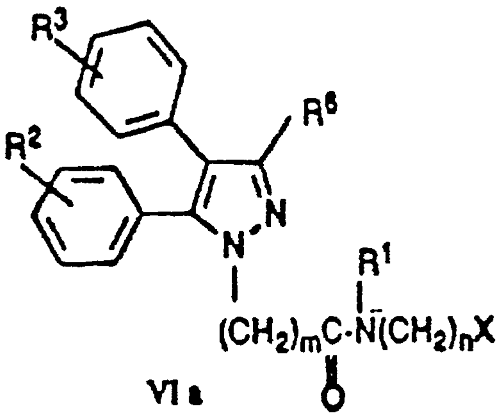

VI a

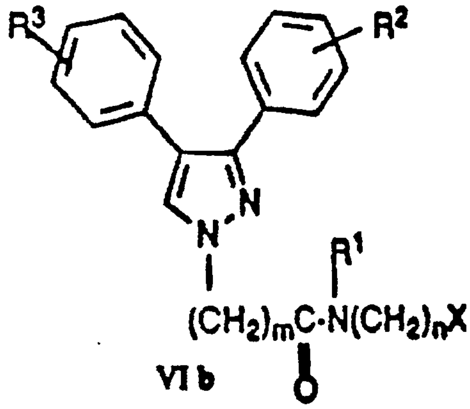

VI b

et on fait réagir ce dernier composé, avec un composé de formule VII :

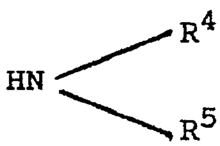

VII

de façon à obtenir le composé correspondant, respectivement de formule Ia ou Ib, dans lequel A représente un groupe $(CH_2)_n$, et $R_1$, $R_2$, $R_3$, $R_4$, $R_5$, m et n sont tels que définis dans la revendication 1, et X représente un groupe susceptible de subir une substitution nucléophile ; et si on le souhaite,

on convertit une base libre obtenue, en un sel d'addition avec un acide de celle-ci.

9. Composition pour le traitement des arythmies cardiaques, comprenant un composé selon l'une quelconque des revendications 1 à 6, selon une quantité efficace pour traiter les arythmies cardiaques, associé avec un ou plusieurs excipients ou diluants pharmaceutiquement acceptables.

10. Utilisation d'un composé selon l'une quelconque des revendications 1 à 7, pour préparer un médicament destiné au traitement des arythmies cardiaques chez un sujet sensible.

**Revendications pour les Etats Contractants; ES, GR**

1. Procédé de préparation d'un composé de formule :

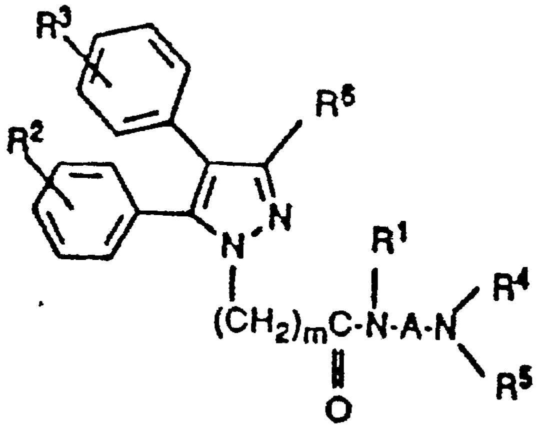

Ia

ou

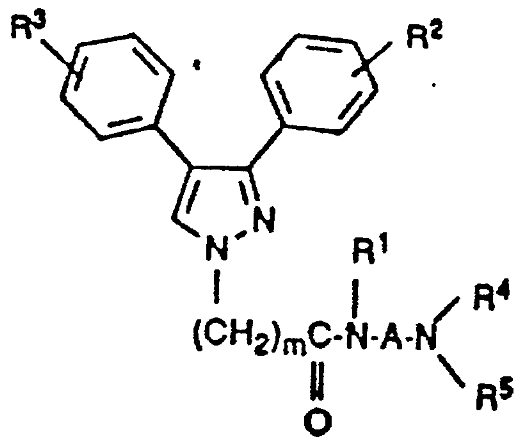

Ib

ou un sel d'addition avec un acide de celui-ci, dans lequel $R^1$ représente un atome d'hydrogène ou un groupe alkyle comportant jusqu'à 4 atomes de carbone ; $R^2$ et $R^3$ représentent indépendamment chacun un atome d'hydrogène, un groupe hydroxy, alkyle comportant jusqu'à 4 atomes de carbone, alkoxy comportant jusqu'à 4 atomes de carbone, ou un atome d'halogène ; $R^4$ et $R^5$ représentent indépendamment chacun un atome d'hydrogène, un groupe alkyle comportant jusqu'à 4 atomes de carbone ou hydroxyalkyle comportant jusqu'à 4 atomes de carbone ; ou $R^4$ et $R^5$ forment ensemble une chaîne alkylène comportant de 4 à 6 atomes de carbone ; $R^6$ représente un atome d'hydrogène ou un groupe hydroxy ; m est égal à un ou deux ; et A représente un groupe $CH_2CH(OH)CH_2$ ou $(CH_2)_n$ dans lequel n est un entier de 2 à 8, caractérisé en ce qu'on fait réagir un composé de formule IIa ou IIb :

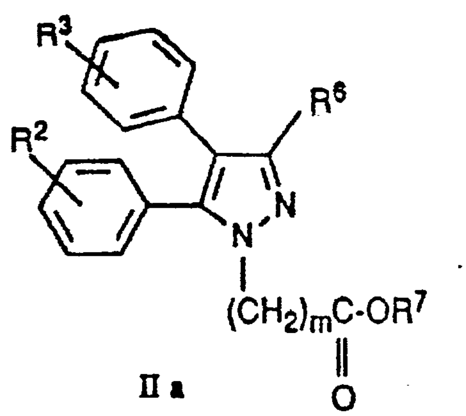

II a

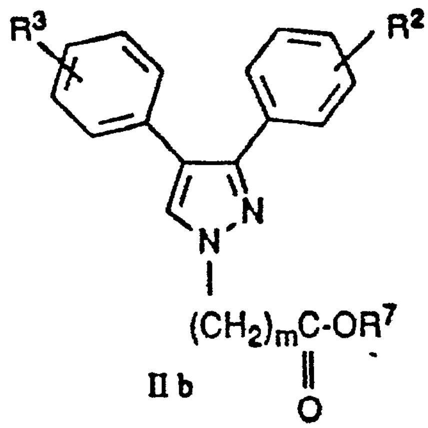

II b

dans laquelle $R^7$ représente un groupe alkyle comportant jusqu'à 4 atomes de carbone, avec : a. un composé de formule III :

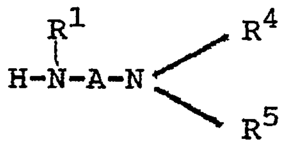

III

pour préparer un composé correspondant, respectivement de formule Ia ou Ib ; ou b. un composé de formule IV :

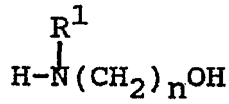

IV

pour préparer un composé correspondant, respectivement de formule Va ou Vb :

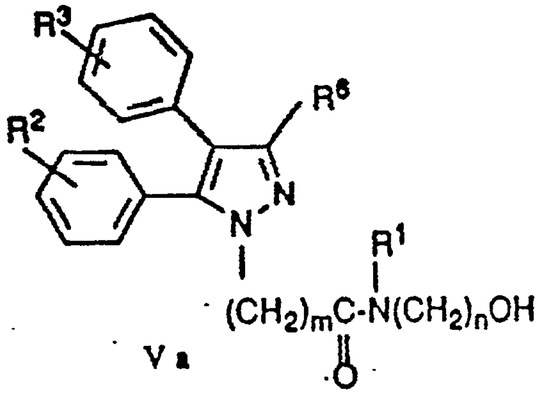

V a

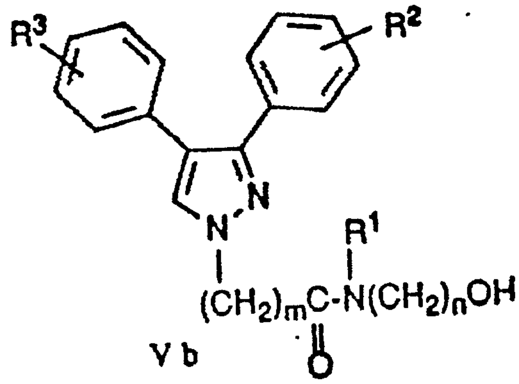

V b

et on fait réagir ce dernier composé obtenu, avec un agent capable de convertir la fonction alcool, en un groupe susceptible de subir une attaque nucléophile, pour préparer un composé correspondant, respectivement de formule VIa ou VIb :

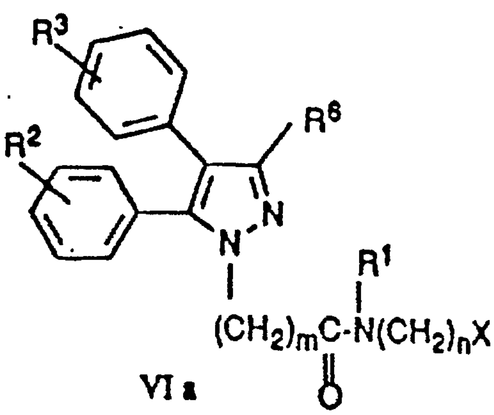

VI a

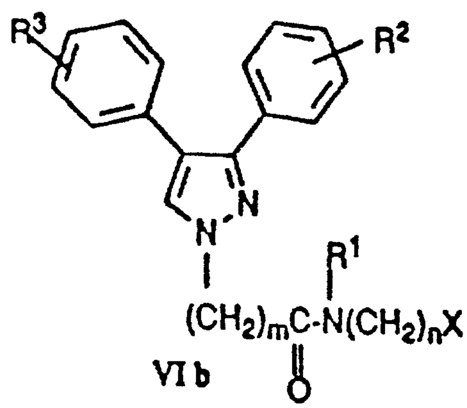

VI b

et on fait réagir ce dernier composé, avec un composé de formule VII :

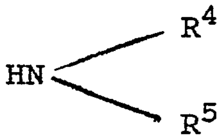

VII

de façon à obtenir le composé correspondant, respectivement de formule Ia ou Ib, dans lequel A représente un groupe $(CH_2)_n$, et $R_1$, $R_2$, $R_3$, $R_4$, $R_5$, m et n sont tels que définis ci-dessus, et X représente un groupe susceptible de subir une substitution nucléophile ; et si on le souhaite,

on convertit une base libre obtenue, en un sel d'addition avec un acide de celle-ci.

2. Procédé selon la revendication 1, selon lequel l'agent b de conversion de la fonction alcool, est un

chlorure d'alkyle ou arylsulfonyle,

X, représentant un groupe $OSO_2R_8$ dans lequel $R_8$ représente un groupe alkyle ou aryle.

3. Procédé selon la revendication 1, selon lequel l'agent b de conversion de la fonction alcool lorsque $R^6$ représente un atome d'hydrogène, est un trihalogénure de phosphore, un pentahalogénure de phosphore, un halogénure de thionyle ou un tétrahalométhane, employé avec une trialkyl phosphine ; le groupe halo correspondant au groupe X, et représentant C1, Br ou I.

4. Procédé selon l'une quelconque des revendications précédentes, dans lequel m est égal un.

5. Procedé selon l'une quelconque des revendications précédentes, dans lequel $R^6$ représente un atome d'hydrogène.

6. Procédé selon l'une quelconque des revendications précédentes, dans lequel A represente un groupe $(CH_2)_n$.

7. Procédé selon la revendication 6, pour préparer le N-[3-diéthylamino)propyl]-4, 5-diphényllH-pyrazole-l-acétamide, dans lequel on faire réagir le 4, 5-diphényl-1H-pyrazole-l-acétate d'éthyle, avec la-3-(diéthylamino)propylamine.

8. Procédé selon la revendication 1, dans lequel m est égal à 2, et A représente un groupe $(CH_2)_n$.